# EUROPEAN PATENT SPECIFICATION

(11) **EP 2 061 452 B1**
(45) Date of publication and mention of the grant of the patent: **26.10.2011**
(21) Application number: 07826053.6
(22) Date of filing: 20.08.2007
(51) Int. Cl.: A61K 31/165, A61K 31/192, A61K 31/216, A61K 31/445, A61K 31/4453, A61K 31/495, A61K 31/506, A61K 31/5375, A61K 45/06, A61P 35/02, A61P 35/00

(54) **ANTI CANCER USE OF CAFFEIC ACID AND DERIVATIVES**
VERWENDUNG VON KAFFEESÄURE UND IHREN DERIVATEN GEGEN KREBS
UTILISATION D'ACIDE CAFÉIQUE ET DE DÉRIVÉS DE CE COMPOSÉ COMME ANTICANCÉREUX

(30) Priority: 01.09.2006 US 842060 P
(43) Date of publication of application: 27.05.2009
(73) Proprietor: Piramal Life Sciences Limited, Mumbai 400 013, Maharashtra (IN)
(72) Inventor: JOSHI, Kalpana, Sanjay, Mumbai 400 063 (IN); SIVAKUMAR, Meenakshi, Mumbai 400 063 (IN); AWARE, Valmik, Sopan, Mumbai 400 063 (IN); WAGH, Vilas, Sampatrao, Mumbai 400 063 (IN); DESHPANDE, Amit, Mumbai 400 063 (IN); SARDE, Ankush, Gangaram, Mumbai 400 063 (IN); SHARMA, Somesh, Mumbai 400 063 (IN)
(74) Representative: Sanderson, Nigel Paul
(86) International application number: PCT/IB2007/053308
(87) International publication number: WO 2008/026125

(56) References cited:
- WO-A-03/101446
- WO-A-2005/099721
- US-A1- 2004 006 138
- US-A1- 2005 282 892
- US-A1- 2007 161 704
- O'HARE THOMAS ET AL: "In vitro activity of Bcr-Abl inhibitors AMN107 and BMS-354825 against clinically relevant imatinib-resistant Abl kinase domain mutants." CANCER RESEARCH 1 JUN 2005, vol. 65, no. 11, 1 June 2005 (2005-06-01), pages 4500-4505, XP002414413 ISSN: 0008-5472 cited in the application
- SAKAGAMI, HIROSHI ET AL: "Effect of methionine depletion on growth and apoptosis in various tumor cell lines" ANTICANCER RESEARCH , 17(4A), 2407-2410 CODEN: ANTRD4; ISSN: 0250-7005, 1997, XP008093697
- SNYDER, DAVID S. ET AL: "Antiproliferative effects of lipoxygenase inhibitors on malignant human hematopoietic cell lines" EXPERIMENTAL HEMATOLOGY (NEW YORK, NY, UNITED STATES) , 17(1), 6-9 CODEN: EXHMA6; ISSN: 0301-472X, 1989, XP008093700

## Description

### FIELD OF THE INVENTION

The present invention relates to the use of a derivative of caffeic acid or a salt thereof represented by general formula (I) as is defined in claim 1 in the treatment of chronic myeloid leukemia (CML) that is resistant to treatment with Gleevec (Glivec, Imatinib mesylate or STI571). The present invention also relates pharmaceutical compositions (for the manufacture of the medicament) including a derivative of caffeic acid or a salt thereof represented by the general formula (1) as is defined in claim 1 for the treatment of chronic myeloid loukemia (CML) that is resistant to treatment with Gleevec

### BACKGROUND OF THE INVENTION

Chronic myeloid leukemia (CML) is characterized by the clonal proliferation of malignant myeloid progenitor cells resulting in excessive number of myeloid cells in all stages of maturation. Development of CML is associated with a specific chromosomal translocation known as the Philadelphia (Ph) chromosome. Somatic mutation in Ph chromosome originates from the reciprocal translocation between the long arms of chromosomes 9 and 22. A molecular consequence of this translocation is the generation of a fusion protein Bcr-Abl, a constitutively activated tyrosine kinase that is detectable throughout the course of the disease. The Ph chromosome produces an enzyme that plays a central role in aberrant cell growth and division. The enzyme, a fusion protein (Bcr-Abl) that enhances tyrosine kinase activity, changes the cell's normal genetic instructions. This aberrant enzyme sends out signals through multiple pathways within the cell, resulting in the overproduction of white blood cells in the body. The result is that, while a healthy cubic millimetre of blood contains 4,000 to 10,000 white blood cells, blood from a patient with CML contains 10 to 25 times this amount. The massive increase in the number of white blood cells characterises CML. In addition to CML, a subset acute lymphoid leukemia (ALL) and acute myeloid Leukemia AML cases are Ph positive leukemias. Gleevec (Glivec, Imatinib mesylate or STI571), an Abl kinase inhibitor is now the first line treatment for patients with CML. Gleevec is indicated for the treatment of patients with Philadelphia chromosome-positive CML in the chronic phase, accelerated phase or in blast crisis. In some cases Gleevec is used after failure of interferon-alpha therapy. So also, in some cases interferon=alpha therapy treatment is given after failure of Gleevec therapy. The effectiveness of Gleevec in CML is based on overall hematologic and cytogenetic response rates. Gleevec is a novel therapy that offers a new treatment option to patients suffering from CML, a disease that previously had limited treatment options. It also has provided researchers with new insights into the biological mechanisms of cancer.

The majority of patients treated with Gleevec experience adverse events at some time. Most events are of mild to moderate grade, but in the Phase II clinical trials for the CML, the drug was discontinued for adverse events in 2% of patients in chronic phase, 3% in accelerated phase and 5% in blast crisis. The most common side effects included nausea, fluid retention, vomiting, diarrhea, hemorrhage, muscle cramps, skin rash, fatigue, headache, dyspepsia and dyspnea, as well as neutropenia and thrombocytopenia. Serious and severe side effects, such as hepatotoxicity (1% to 4%), fluid retention syndrome (3% to 12%), neutropenia (8% to 48%) and thrombocytopenia (less than 1 % to 33%) have also been reported in some patients. There is no long-term safety data on Gleevec treatment available up to now. Although the majority of CML patients treated with Gleevec show significant hematological and cytogenetic responses, resistance to treatment with Gleevec is still a problem, mainly in patients in the accelerated or blast crisis phases of the disease.

A review article in Leukemia (2004, 1-11), a Nature publication, describes the possible reasons for development of resistance to treatment with Gleevec. Resistance to treatment with Gleevec in patients has been associated with a heterogeneous array of mechanisms that range from nonspecific multi-drug resistance to Bcr-Abl inherent genetic alterations. The most frequently identified mechanism of acquired Gleevec resistance is Bcr-Abl kinase domain point mutations that impair Gleevec binding either by interfering with a Gleevec binding site or by stabilizing a Bcr-Abl conformation with reduced affinity to Gleevec. US20060057157A1 also describes the possible reason for development of resistance to treatment with Gleevec. According to the reference, induction of apoptosis is the principal mechanism by which the majority of chemotherapeutic agents exert their function. Consequently, failure to undergo apoptosis is the likely mechanism mediating drug resistance in tumors.

Human T315I is the most commonly observed mutated form of Bcr-Abl, which is Gleevec resistant. T315I is result of point mutation of threonine residue at 315 position to isoleucine in the kinase domain of Bcr-Abl protein.

Blood, 2003, 101, 690-698, describes K-562-R resistant cell line. K-562 is one of the human leukemic cell lines which contains a wild type Bcr-Abl protein, while K-562-R is a K-562 cell line which is made resistant to Gleevec by continuous exposure to Gleevec (2 µg/ml) for several passages.

Hematology, 2005, 183-187, describes two investigational small molecule ABL kinase inhibitors, Dasatinib (BMS-354825) and AMN107, which have exhibited efficacy in phase I clinical trials for the treatment of imatinib mesylate (Gleevec) - resistant CML, and are being further evaluated clinically. The long term efficacy of these new inhibitors remains to be determined.

Cancer Res., 2006, 66(2), 1007-1014, and Proceedings of the National Academy of Sciences (PNAS), 2005, 102(6), 1992-1997 describe two compounds VX-680 and ON-012380 active against Gleevec resistant CML (T315I).

Cancer Res., 2005, 65 (11), 4500-4505 describes *in-vitro* activity of Bcr-Abl inhibitors AMN107 and BMS-354825 against clinically relevant imatinib mesylate-resistant Abl Kinase domain mutants. The article describes various imatinib mesylate resistant cell lines- Ba/F3 Bcr-Abl/T315I, Ba/F3 Bcr-Abl/E255K, Ba/F3 Bcr-Abl/H396P, Ba/F3 Bcr-Abl/M351T, Ba/F3 Bcr-Abl/F359V, Ba/F3 Bcr-Abl/E255V, Ba/F3 Bcr-Abl/F317L, Ba/F3 Bcr-Abl/H396R, Ba/F3 Bcr-Abl/M244V, Ba/F3 Bcr-Abl/Q252H, Ba/F3 Bcr-Abl/Y253F and Ba/F3 Bcr-Abl/Y253H.

The phytochemical caffeic acid (3,4-dihydroxy cinnamic acid) is a constituent of coffee, fruits vegetables, grains and honeybee propolis. Caffeic acid is known to have various pharmacological activities such as antioxidant and antiviral effects. Caffeic acid or its derivatives such as esters are reported to have anti cancer activity. Following reports indicate the specific anti cancer properties of caffeic acid or its derivatives.

Breast Cancer Res., 2004, 6, R63-R74, describes anti-proliterative and apoptotic effects of caffeic acid on T47D human breast cancer cells.

Bull. Korean Chem., 2001, 22(10), 1131-1135, describes the inhibitory effect of caffeic acid methyl ester on Fos-Jun-DNA complex formation and suppression of cancer cell growth. The publication describes the Cytotoxic effect against human cancer cell line (A549 and K-562).

Biol. Pharm. Bull., 2005, 28(12), 2338-2341, describes the growth inhibition by octylcaffeate, of human histiolytic lymphoma U937 cells via apoptosis.

Cancer Letters, 1996, 108, 211-214, describes the inhibitory effect of caffeic acid phenethyl ester on human Leukemia HL-60 cells.

J. Nutritional Biochemistry, 2006, 17(5), 356-362, describes the inhibitory effects of caffeic acid phenethyl ester on cancer cell metastasis mediated by the down-regulation of matrix metalloproteinase expression in human HT1080 fibrosarcoma cells.

The following patent applications disclose compositions containing caffeic acid or its derivatives.

WO9105543 describes pharmaceutical or cosmetic composition containing caffeic acid or one of its esters or arnides as active ingredient.

JP60013712 describes drug containing caffeic acid methyl ester as an active ingredient, for the inhibition of 5-lipoxygenase activity.

US 2004/0006138 and US 2005/0282892 describe pharmaceutical compositions comprising analogues of chlorogenic acid and/or its salts for use in the treatment of chronic myeloid leukemia where Bcr-Abl kinase is constitutively expressed in animals and humans.

There is an urgent need for medicaments for treating chronic myeloid leukemia (CML) that is resistant to treatment with Gleevec due, for example, to the Bcr-Abl mutation.

### SUMMARY OF THE INVENTION

The present invention relates to a derivative of caffeic acid or a salt thereof represented by general formula (I) as is defined in claim 1 for use in treating chronic myeloid leukemia (CML) that is resistant to treatment with Gleevec.

Compounds of the present invention can be employed in inhibiting growth of Gleevec resistant cell lines including K-562-R and 32Dcl^{Bcr-Abl T315I}, Ba/F3 Bcr-Abl/T315I, Ba/F3 Bcr-Abl/E255K, Ba/F3 Bcr-Abl/H396P, Ba/F3 Bcr-Abl/M351T, Ba/F3 Bcr-Abl/F359V, Ba/F3 Bcr-Abl/E255V, Ba/F3 Bcr-Abl/F317L, Ba/F3 Bcr-Abl/H396R, Ba/F3 Bcr-Abl/M244V, Ba/F3 Bcr-AblQ252H, Ba/F3 Bcr-Abl/Y253F, and Ba/F3 Bcr-Abl/Y253H.

A pharmaceutical composition for treating chronic myeloid leukemia (CML) that is resistant to treatment with Gleevec, which includes a therapeutically effective amount of caffeic acid or a derivative of formula (1), or a salt thereof is also describes.

The use of caffeic acid or a derivative or a salt thereof for the manufacture of a medicament for treating chronic myeloid leukemia (CML) that is resistant to treatment with Gleevec is also described.

### BRIEF DESCRIPTION OF THE DRAWINGS

**Figure 1**: Illustrates anti-proliferative activity of compounds of the present invention in imatinib mesylate-sensitive (Ba/F3 Bcr-Abl/WT) and imatinib mesylate-resistant (Ba/F3 Bcr-Abl/T315I) cell lines.
**Figure 2**: Illustrates anti-proliferative activity of compounds of the present invention, expressed as mean IC₅₀ values in µM, in cells with imatinib mesylate resistant low frequency mutations (Ba/F3 Bcr-Abl/E255K, Ba/F3 Bcr-Abl/H396P, Ba/F3 Bcr-Abl/E255V, Ba/F3 Bcr-Abl/M244V and Ba/F3 Bcr-Abl/Q252H) as seen in the clinic.
**Figure 3**: Illustrates anti-proliferative activity of compounds of the present invention, expressed as mean IC₅₀ values in µm, in cells with imatinib mesylate resistant high frequency mutations (Ba/F3 Bcr-Abl/T315I, Ba/F3 Bcr-Abl/M351T, Ba/F3 Bcr-Abl/F359V, Ba/F3 Bcr-Abl/F317L, Ba/F3 Bcr-Abl/H396R, Ba/F3 Bcr-Abl/Y253F and Ba/F3 Bcr-Abl/Y253H) as seen in the clinic.
**Figure 4**: Illustrates induction of apoptosis after 48 hours, by compounds of the present invention (at 5 µM), in imatinib mesylate sensitive and resistant cell lines.
**Figure 5A**: Illustrates *in-vivo* efficacy of compounds of the present invention in SCID mice in imatinib mesylate resistant (Ba/F3 Bcr-Abl/T315I) xenograft model.
**Figure 5B**: Illustrates *in-vivo* efficacy of compounds of the present invention in SCID mice in imatinib mesylate sensitive (Ba/F3 Bcr-Abl/WT) xenograft model.

### DETAILED DESCRIPTION OF THE INVENTION

The present invention provides derivatives of caffeic acid represented by the following general formula (1) 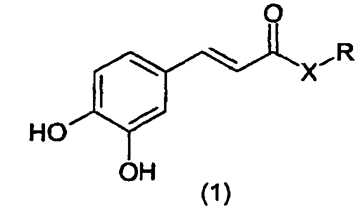 wherein
X is O, NH, or heterocyclyl;
R is a straight chain or branched alkyl which is optionally substituted, aryl which is optionally substituted, heterocyclyl which is optionally substituted, or absent;
in all its stereoisomeric and tautomeric forms, and mixtures thereof in all ratios, a pharmaceutically acceptable salt thereof, for use in the treatment of chronic myeloid leukemia (CML) that is resistant to treatment with Gleevec (Imatinib mesylate).

### Definitions

Listed below are definitions, which apply to the terms as they are used throughout the specification and the appended claims.

As used herein, the term "alkyl" refers to an aliphatic group, including straight or branched-chain alkyl groups. Furthermore, unless stated otherwise, the term "alkyl" includes unsubstituted alkyl groups as well as alkyl groups that are substituted by one or more different substituents.

In an embodiment, a straight chain or branched chain alkyl has 20 or fewer carbon atoms in its backbone (e.g., C₁-C₂₀ for straight chain, C₃-C₂₀ for branched chain), for example, 15 or fewer carbon atoms. Examples of alkyl residues containing from 1 to 20 carbon atoms are: methyl, ethyl, propyl, butyl, pentyl, hexyl, heptyl, octyl, nonyl, decyl, undecyl, dodecyl, tetradecyl, hexadecyl, octadecyl and eicosyl, the n-isomers of all these residues, isopropyl, isobutyl, 1-methylbutyl, isopentyl, neopentyl, 2,2-dimethylbutyl, 2-methylpentyl, 3-methylpentyl, isohexyl, 2,3,4-trimethylhexyl, isodecyl, sec-butyl, or tert-butyl. Suitable alkyl residues contain from 1 to 6 carbon atoms, for example, from 1 to 4 carbon atoms, such as methyl, ethyl, propyl, isopropyl, n-propyl, t-butyl, n-butyl, sec-butyl, or iso-butyl.

The alkyl groups may be substituted or unsubstituted. Unless stated otherwise, alkyl groups can be unsubstituted or substituted by one or more (for example 1, 2, 3, 4 or 5) identical or different substituents. Any kind of substituent present in substituted alkyl residues can be present in any desired position provided that the substitution does not lead to an unstable molecule. A substituted alkyl refers to an alkyl residue in which one or more, for example, 1, 2, 3, 4 or 5 hydrogen atoms are replaced with substituents, for example, alkyl, halogen, hydroxyl, carbonyl, carboxyl, alkoxyl, cycloalkyl, ester, ether, cyano, amino, mono- or di-alkylamino, amido, imino, nitro, aralkyl, acyloxy, heterocyclyl, aryl, or heteroaryl.

As used herein the term "aryl" refers to a monocyclic or polycyclic hydrocarbon group having up to 14 ring carbon atoms, for example, up to 10 ring carbon atoms, in which at least one carbocyclic ring is present that has a conjugated π electron system. Suitable examples of (C₆-C₁₄)-aryl residues include phenyl, naphthyl, biphenyl, fluorenyl or anthracenyl, especially phenyl and naphthyl. Unless stated otherwise aryl residues, for example phenyl, naphthyl or fluorenyl, can in general be substituted or unsubstituted by one or more substituents, for example, up to five identical or different substituents selected from the group consisting of halogen, alkyl, fluoroalkyl, hydroxyl, alkoxyl, aryloxy, amino, cyano, nitro, thiol, imine, amide, carbonyl, aryl, and heterocyclyl.

The term "heterocyclyl" refers to a saturated, partially unsaturated or aromatic monocyclic or polycyclic ring system containing up to 14 ring atoms of which 1, 2 or 3 are identical or different heteroatoms selected from: nitrogen, oxygen, or sulfur. The heterocyclyl group may, for example, have 1 or 2 oxygen atoms and/or 1 or 2 sulfur atoms and/or 1 to 4 nitrogen atoms in the ring. The ring heteroatoms can be present in any desired number and in any position with respect to each other provided that the resulting heterocyclic system is known in the art and is stable and suitable as a subgroup in a drug substance. Suitable examples of heterocyclyl groups include piperazinyl, piperidinyl imidazolyl, pyrrolidinyl, morpholinyl, benzoxazolyl, quinolyl, isoquinolyl, carbazolyl, indolyl, isoindolyl, phenoxazinyl, benzothiazolyl, benzimidazolyl, benzoxadiazolyl, or benzofurazanyl.

The heterocyclyl groups may be substituted or unsubstituted. Unless stated otherwise, and irrespective of any substituents bonded to heterocyclyl groups in the definition of the compounds of formula 1, the heterocyclyl group can be unsubstituted or substituted on ring carbon atoms with one or more substituents, up to five identical or different substituents. Suitable examples of substituents for the ring carbon and ring nitrogen atoms include: alkyl, aralkyl, alkoxyl, halogen, hydroxyl, hydroxyalkyl, fluoroalkyl, aryloxyl, amino, cyano, nitro, thiol, imine, amide, carbonyl, alkylcarbonyl, arylcarbonyl, aryl, or heterocyclyl. The substituents can be present at one or more positions provided such that a stable molecule results.

As used herein the term "aralkyl" refers to an alkyl group substituted with an aryl or heteroaryl group, wherein the terms alkyl, aryl and heteroaryl are as defined above. Exemplary aralkyl groups include -(CH₂)ₚ-phenyl or -(CH₂)ₚ-pyridyl, wherein p is an integer from 1 to 3. The aralkyl group may be further substituted with alkyl, hydroxy, halogen, cyano, nitro, amino, aryl, heteroaryl, or the like.

The term "halogen" refers to fluorine, chlorine, bromine, or iodine.

As used herein, the terms "treatment" "treat" and "therapy" and the like refer to alleviate, slow the progression, prophylaxis, attenuation or cure of existing disease (e.g., chronic myeloid leukemia (CML) that is resistant to treatment with Gleevec). "Prevent", as used herein, refers to delaying, slowing, inhibiting, reducing or ameliorating the onset of chronic myeloid leukemia (CML) that is resistant to treatment with Gleevec.

As used herein, the term "pharmaceutically acceptable" means that the carrier, diluent, excipients, and/or salt must be compatible with the other ingredients of the formulation, and not deleterious to the recipient thereof.

The term, "therapeutically effective amount" as used herein means an amount of compound or composition (e.g., caffeic acid or derivative of formula (1)) sufficient to significantly induce a positive modification in the condition to be regulated or treated, but low enough to avoid side effects if any (at a reasonable benefit/risk ratio), within the scope of sound medical judgment. The therapeutically effective amount of the compound or composition will vary with the particular condition being treated, the age and physical condition of the end user, the severity of the condition being treated/prevented, the duration of the treatment, the nature of concurrent therapy, the specific compound or composition employed, the particular pharmaceutically acceptable carrier utilized, and like factors.

### Caffeic Acid Compounds and Methods Employing Them

Caffeic acid or derivatives of general formula (1) are known in the literature and may be synthesized by scheme I given below, and may optionally be converted to their pharmaceutically acceptable salts.

3,4-Dihydroxybenzaldehyde **(A)** can be converted to piperonal **(B)** by treatment with dibromomethane in the presence of cesium carbonate in dry N,N-dimethylformamide by the method described in Tetrahedron 2003, 59, 4383 - 4387. Piperonal **(B)**, thus obtained, may be subjected to Knoevenagel condensation with malonic acid to yield 3,4-(methylenedioxy)cinnamic acid **(C)**, by the method described in Eur. J. Med. Chem. 2002, 37, 979 - 984. The acid **(C)** may be converted to its methyl ester **(D)** by reaction with thionyl chloride and methanol. The deprotection of the methylenedioxy group of methyl ester **(D)** using boron tribromide may be carried out by the procedure described in J. Org. Chem. 1974, 39, 1427 -1429. The work up of the reaction mixture would result in the hydrolysis of the ester to yield caffeic acid **(E)**. Caffeic acid **(E)** can be converted to an ester **(F)** (X = O and R = alkyl, aryl, or heterocyclyl) by treatment with thionyl chloride or oxalyl chloride in presence of a suitable alcohol at 0 °C followed by allowing the mixture to warm to room temperature or by heating at reflux, as per the requirement of the reaction. Caffeic acid **(E)** can be converted to an amide **(F)** (X = NH and R = H, alkyl, aryl or heterocyclyl) by coupling with a suitable amine utilizing 1-(3-dimethylaminopropyl)-3-ethyl carbodiimide hydrochloride (EDC), 1-hydroxy benzotriazole (HOBt), and 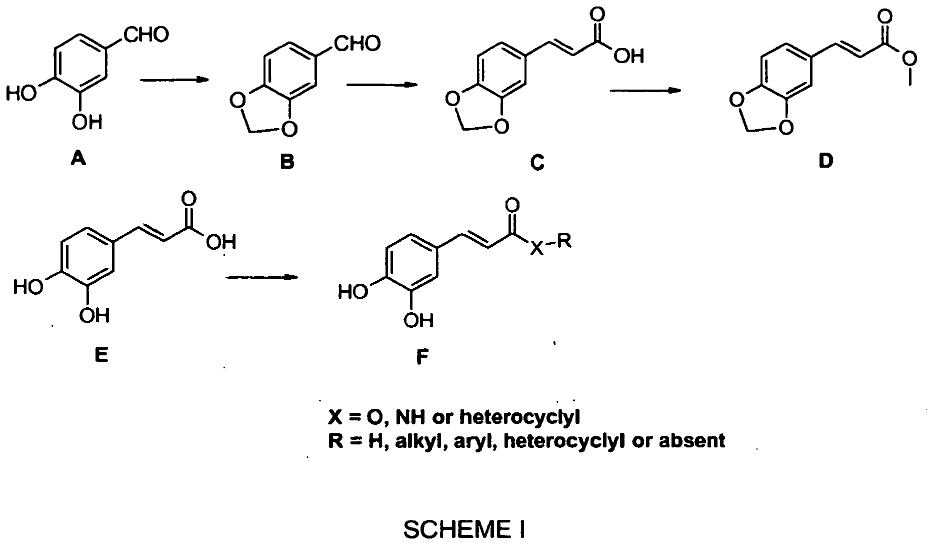 triethylamine in methylene chloride at room temperature by the procedure described in Bioorg. Med. Chem. Lett. 2004, 14, 4677 - 4681. In an alternative method, caffeic acid can be converted to its acid chloride by treatment with thionyl chloride or oxalyl chloride which can be subsequently converted to an amide **(F)** (X = NH and R = H, alkyl, aryl or heterocyclyl) by treatment with the amine of choice.

Caffeic acid or derivatives of general formula (1) can also be obtained from natural sources such as plant extracts.

Derivatives of caffeic acid of formula (1) of the present invention are useful for treating chronic myeloid leukemia (CML) that is resistant to treatment with Gleevec.

The antiproliferative activity of the compounds of formula (1) can be evaluated against Gleevec resistant mutated cell lines such as K-562-R and 32Dcl^{Bcr-Abl T315I}. Several other hematopoietic cell lines can also be used to study the compounds for antiproliferative activity. The cell lines include Ba/F3 Bcr-Abl/T315I, Ba/F3 Bcr-Abl/E255K, Ba/F3 Bcr-Abl/H396P, Ba/F3 Bcr-Abl/M351T, Ba/F3 Bcr-Abl/F359V, Ba/F3 Bcr-Abl/E255V, Ba/F3 Bcr-Abl/F317L, Ba/F3 Bcr-Abl/H396R, Ba/F3 Bcr-Abl/M244V, Ba/F3 Bcr-Abl/Q252H, Ba/F3 Bcr-Abl/Y253F, or Ba/F3 Bcr-Abl/Y253H. An aspect of this invention is the treatment of a mammal (e.g., a human) suffering from chronic myeloid leukemia (CML) that is resistant to treatment with Gleevec by administering a therapeutically effective amount of a compound of the general formula (1) or a pharmaceutically acceptable salt of the compound to the mammal.

Also disclosed is the prevention, reduction or the minimizing of damage resulting from chronic myeloid leukemia (CML) that is resistant to treatment with Gleevec by administering to an affected mammal (e.g., a female or male human) a therapeutically effective amount of a compound of the general formula (1) or a pharmaceutically acceptable salt of the compound.

Representative compounds of formula (1) useful in the treatment of chronic myeloid leukemia (CML) that is resistant to treatment with Gleevec in accordance with the present invention include:
3-(3,4-Dihydroxy-phenyl)-acrylic acid methyl ester;
3-(3,4-Dihydroxy-phenyl)-acrylic acid ethyl ester;
(*E*)-3-(3,4-dihydroxyphenyl)-acrylic acid 2-nitro-ethyl ester;
3-(3,4-Dihydroxy-phenyl)-acrylic acid *n*-propyl ester;
3-(3,4-Dihydroxy-phenyl)-acrylic acid *i*-propyl ester;
3-(3,4-Dihydroxy-phenyl)-acrylic acid butyl ester;
(*E*)-3-(3,4-dihydroxy-phenyl)-1-piperidin-1-yl-propenone;
3-(3,4-Dihydroxy-phenyl)-1-(4-ethyl-piperazin-1-yl)-propenone;
1-(4-Benzyl-piperazin-1-yl)-3-(3,4-dihydroxy-phenyl)-propenone;
(*E*)-3-{4-[3-(3,4-dihydroxy-phenyl)-acryloyl]-piperazin-1-yl} propionitrile;
(*E*)-3-(3,4-dihydroxyphenyl)-1-[4-(1-methyl-piperidin-4-yl) piperazin-1-yl]- propenone;
(*E*)-3-(3,4-dihydroxyphenyl)-1-(4-phenylpiperazin-1-yl)-propenone;
*(E*)-1-(4-acetyl-piperazin-1-yl)-3-(3,4-dihydroxy-phenyl)-propenone;
(*E*)-3-(3,4-dihydroxy-phenyl)-1-(4-phenethyl-piperazin-1-yl)-propenone;
(*E*)-3-(3,4-dihydroxy-phenyl)-1-morpholin-4-yl-propenone;
3-(3,4-dihydroxy-phenyl)-N-(3-dimethylamino-propyl)-acrylamide;
(*E*)-3-(3,4-dihydroxyphenyl)-N-isopropyl-acrylamide; or
1-(4-Benzyl-piperidin-1-yl)-3-(3,4-dihydroxy-phenyl)-propenone.

Suitable compounds for the treatment chronic myeloid leukemia (CML) that is resistant to treatment with Gleevec in accordance with the present invention include:
3-(3,4-Dihydroxy-phenyl)-acrylic acid methyl ester;
3-(3,4-Dihydroxy-phenyl)-acrylic acid ethyl ester;
3-(3,4-Dihydroxy-phenyl)-acrylic acid *n*-propyl ester;
3-(3,4-Dihydroxy-phenyl)-acrylic acid *i*-propyl ester;
1-(4-Benzyl-piperazin-1-yl)-3-(3,4-dihydroxy-phenyl)-propenone;
(*E*)-3-(3,4-dihydroxyphenyl)-N-isopropyl-acrylamide; or
1-(4-Benzyl-piperidin-1-yl)-3-(3,4-dihydroxy-phenyl)-propenone.

Also described are pharmaceutical compositions that contain an effective amount of at least one compound of the general formula (1) or its physiologically tolerable salt in addition to a customary pharmaceutically acceptable carrier, and a process for the production of a pharmaceutical, which includes bringing at least one compound of formula (1), into a suitable administration form using a pharmaceutically suitable and physiologically tolerable excipient and, if appropriate, further suitable active compounds, additives or auxiliaries.

The compounds of the present invention are useful for treating cancer, particularly for chronic myeloid leukemia (CML) that is not responding to Gleevec treatment. The present invention accordingly relates to the use of a compound of the general formula (1) for the manufacture of a medicament for the treatment of chronic myeloid leukemia (CML) that is not responding to Gleevec treatment.

The present invention also envisages the use of a compound of the general formula (1) or a pharmaceutically acceptable salt of the compound in combination with other pharmaceutically active compounds. For instance, a pharmaceutical composition including a compound of the general formula (1) or a pharmaceutically acceptable salt can be administered to a mammal, in particular a human, with any other compound active against Gleevec resistant cells or tumors, or any other pharmaceutically active compound known to be useful in treating one of the above mentioned disorders, in mixtures with one another or in the form of pharmaceutical preparations.

The compounds of the present invention can be used in a method for reducing the population of Gleevec sensitive (e.g., K-562 or Ba/F3 Bcr-Abl/WT) and Gleevec resistant (K-562-R and 32Dcl^{Bcr-Abl T315I}, Ba/F3 Bcr-Abl/T315I, Ba/F3 Bcr-Abl/E255K, Ba/F3 Bcr-Abl/H396P, Ba/F3 Bcr-Abl/M351T, Ba/F3 Bcr-Abl/F359V, Ba/F3 Bcr-Abl/E255V, Ba/F3 Bcr-Abl/F317L, Ba/F3 Bcr-Abl/H396R, Ba/F3 Bcr-Abl/M244V, Ba/F3 Bcr-Abl/Q252H, Ba/F3 Bcr-Abl/Y253F, or Ba/F3 Bcr-Abl/Y253H) chronic myeloid leukemia (CML) cells *in-vitro.*

The *in-vivo* efficacy of the compounds of the present invention in Gleevec-sensitive and Gleevec-resistant tumor models can be evaluated by using cell lines such as Ba/F3 transfectants expressing full-length wild type Bcr-Abl (Ba/F3 Bcr-Abl/WT) or mutated Bcr-Abl (Ba/F3 Bcr-Abl/T315I) in xenograft models of SCID (Severely Combined Immune-Deficient) mice.

The present invention accordingly relates to the use of a compound of the general formula (1) for the manufacture of a medicament for the treatment of chronic myeloid leukemia (CML) that is not responding to Gleevec treatment.

In an aspect of the invention, the treatments described herein use the pharmaceutical compositions described above can be administered by the following administration routes, modes, etc.

### Pharmaceutical Compositions and Methods

The pharmaceuticals can be administered orally, for example in the form of pills, tablets, coated tablets, capsules, granules or elixirs. Administration, however, can also be carried out rectally, for example in the form of suppositories, or parenterally, for example intravenously, intramuscularly or subcutaneously, in the form of injectable sterile solutions or suspensions, or topically, for example in the form of solutions or transdermal patches, or in other ways, for example in the form of aerosols or nasal sprays.

The pharmaceutical preparations according to the invention are prepared in a manner known and familiar to one skilled in the art. Pharmaceutically acceptable inert inorganic and/or organic carriers and/or additives can be used in addition to the compound(s) of the general formula (1), and/or its (their) physiologically tolerable salt(s). For the production of pills, tablets, coated tablets and hard gelatin capsules it is possible to use, for example, lactose, corn starch or derivatives thereof, gum arabica, magnesia or glucose, etc. Carriers for soft gelatin capsules and suppositories are, for example, fats, waxes, natural or hardened oils, etc. Suitable carriers for the production of solutions, for example injection solutions, or of emulsions or syrups are, for example, water, physiological sodium chloride solution or alcohols, for example, ethanol, propanol or glycerol, sugar solutions, such as glucose solutions or mannitol solutions, or a mixture of the various solvents which have been mentioned.

The pharmaceutical preparations normally contain about 1 to 99 %, for example, about 5 to 70%, or from about 10 to about 30 % by weight of the compound of the formula (1) or its physiologically tolerable salt. The amount of the active ingredient of the formula (1) or its physiologically tolerable salt in the pharmaceutical preparations normally is from about 5 to 500 mg. The dose of the compounds of this invention, which is to be administered, can cover a wide range. The dose to be administered daily is to be selected to suit the desired effect. A suitable dosage is about 0.001 to 100 mg/kg/day of the compound of formula (1) or their physiologically tolerable salt, for example, about 0.01 to 50 mg/kg/day of a compound of formula (1) or a pharmaceutically acceptable salt of the compound. If required, higher or lower daily doses can also be administered. Actual dosage levels of the active ingredients in the pharmaceutical compositions of this invention may be varied so as to obtain an amount of the active ingredient, which is effective to achieve the desired therapeutic response for a particular patient, composition, and mode of administration without being toxic to the patient.

The selected dosage level will depend upon a variety of factors including the activity of the particular compound of the present invention employed, or the ester, salt or amide thereof, the route of administration, the time of administration, the rate of excretion of the particular compound being employed, the duration of the treatment, other drugs, compounds and /or materials used in combination with the particular compounds employed, the age, sex, weight, condition, general health and prior medical history of the patient being treated, and like factors well known in the medical arts.

In addition to the active ingredient, the compound of the general formula (1) or its physiologically acceptable salt and carrier substances, the pharmaceutical preparations can contain additives such as, for example, fillers, antioxidants, dispersants, emulsifiers, defoamers, flavors, preservatives, solubilizers or colorants. They can also contain two or more compounds of the general formula (1) or their physiologically tolerable salts. Furthermore, in addition to at least one compound of the general formula (1) or its physiologically tolerable salt, the pharmaceutical preparations can also contain one or more other therapeutically or prophylactically active ingredients.

It is understood that modifications that do not substantially affect the activity of the various embodiments of this invention are included within the invention disclosed herein. Accordingly, the following examples are intended to illustrate the present invention.

### EXAMPLES

### Example 1 (reference)

### 3-(3,4-Dihydroxy-phenyl)-acrylic acid [caffeic acid] (Compound 1)

Compound of Example 1c (6.0 g, 0.029 mol) was dissolved in chloroform (20 mL), to which a solution of 25 % w/v boron tribromide in chloroform (90 mL) was added and stirred at 25 °C for 16 h. The reaction mixture was quenched by dropwise addition of 10 % aqueous sodium bicarbonate solution, at 25 °C and stirring, to achieve final pH 7. The organic layer was separated, washed with water (100 mL), brine (100 mL), dried over anhydrous sodium sulfate and concentrated under reduced pressure to obtain the title compound.
Yield: 2.0 g (38.3 %). ¹H NMR (CDCl₃ + DMSO-d₆): δ 6.83 (d, 1H, J = 15.6 Hz), 6.41 (d, 1 H), 6.25 (dd, 1 H), 6.18 (d, 1 H), 6.52 (d, 1 H, J = 15.6 Hz).

### Example 1a

### Benzo[1,3]dioxole-5-carbaldehyde [piperonal]

3,4-Dihydroxy benzaldehyde (25 g, 0.181 mol), cesium carbonate (88.29 g, 0.271 mol) and dibromomethane (19.0 mL, 0.271 mol) were dissolved in N,N-dimethylformamide (400 mL) and the reaction mixture was heated to 110 °C for 1.5 h. N,N-dimethylformamide was evaporated under reduced pressure, and the reaction mixture was diluted using ethyl acetate (500 mL), washed with water (2 x 250 mL) and brine (2 x 100 mL). The organic layer was dried over anhydrous sodium sulfate and concentrated under reduced pressure to obtain the title compound.
Yield: 23.0 g (84.6 %). ¹H NMR (CDCl₃): δ 9.8 (s, 1 H), 7.42 (d, 1 H), 7.34 (d, 1 H), 6.94 (d, 1 H), 6.09 (s, 2H).

### Example 1b

### 3-Benzo[1,3]dioxol-5-yl-acrylic acid

A mixture of compound of Example 1 a (23 g, 0.153 mol) and malonic acid (31.77 g, 0.305 mol) were dissolved in pyridine (69 mL) with stirring and piperidine (0.92 mL) was added. The reaction mixture was heated at 85 °C for 1 h, after which the temperature was further increased to 105 °C and maintained at this temperature for 3 h. The reaction mixture was cooled, diluted with water (50 mL) and made basic (to pH 9) using 10% aqueous sodium hydroxide solution. The mixture was extracted with ethyl acetate (2 x 250 mL). The aqueous layer was made acidic (to pH 2) using 50 % aqueous hydrochloric acid and the resulting solid was filtered, washed with water and dried to obtain the title compound.
Yield: 20.0 g (68 %). ¹H NMR (DMSO-d₆): δ 7.42 (d, 1 H, J = 15.9 Hz), 7.31 (s, 1 H), 7.10 (d, 1 H), 6.92 (d, 1 H), 6.37 (d, 1H, J = 15.9 Hz), 6.04 (s, 2H); MS (ES-) : 191 (M-1).

### Example 1c

### 3-Benzo[1,3]dioxol-5-yl-acrylic acid methyl ester

Compound of Example 1b (25 g, 0.13 mol) was dissolved in methanol (200 mL) and cooled to 0-5 °C. Thionyl chloride (14.2 mL, 0.195 mol) was added dropwise and the mixture was stirred at 0 °C for 30 mins. The reaction mixture was allowed to warm to room temperature (25 °C) and stirred for 16 h. At the end of the reaction, the mixture was concentrated under reduced pressure. The residue was diluted with 10 % sodium bicarbonate to achieve pH 7 and extracted with ethyl acetate (2 x 250 mL). The organic layer was washed with water (100 mL) and brine (100 mL), dried over anhydrous sodium sulfate and concentrated under reduced pressure to obtain the title compound as a white solid.
Yield: 20.0 g (74.6 %). ¹H NMR (DMSO-d₆): δ 7.55 (d, 1 H, J = 15.9 Hz), 7.38 (d, 1 H), 7.17 (dd, 1 H), 6.93 (d, 1 H), 6.48 (d, 1 H, J = 15.9 Hz), 6.05 (s, 2H), 3.68 (s, 3H).

### Example 2

### 3-(3,4-Dihydroxy-phenyl)-acrylic acid methyl ester (Compound 2)

Compound 1 (8.0 g, 0.044 mol) was dissolved in methanol (70 mL) and cooled to 0 °C. To this, thionyl chloride (4.84 mL, 0.066 mol) was added dropwise maintaining the temperature at 0 °C for a period of 30 mins. The reaction mixture was allowed to warm to room temperature (25 °C) and stirred for 16 h. The reaction mixture was concentrated under reduced pressure. The residue was diluted with 10 % sodium bicarbonate to achieve pH 7 and extracted with ethyl acetate (2 x 100 mL). The organic layer was washed with water (50 mL) and brine (50 mL), dried over anhydrous sodium sulfate and concentrated under reduced pressure to obtain the title compound as a white solid.
Yield: 7.5 g (87.8 %). ¹H NMR (DMSO-d₆): δ 7.45 (d, 1 H, J = 15.9 Hz), 7.02 (s, 1 H), 6.98 (d, 1 H), 6.73 (d, 1 H), 6.24 (d, 1 H, J = 15.9 Hz), 3.7 (s, 3H); MS (ES-): 193 (M-1).

### Example 3

### 3-(3,4-Dihydroxy-phenyl)-acrylic acid ethyl ester (Compound 3)

Compound 1 (0.2 g, 1.11 mmol) was dissolved in ethanol (15 mL) and cooled to 0 °C. To this, thionyl chloride (0.1 mL, 1.41 mmol) was added dropwise maintaining the temperature at 0 °C over a period of 30 mins. The reaction mixture was allowed to warm to room temperature (25 °C), heated to 50 °C and maintained at that temperature overnight (16 h). The reaction mixture was concentrated under reduced pressure. The residue was diluted with 10 % sodium bicarbonate to achieve pH 7 and extracted with ethyl acetate (2 x 10 mL). The organic layer was washed with water (5 mL) and brine (5 mL), dried over anhydrous sodium sulfate and concentrated under reduced pressure. The residue was purified by flash chromatography (silica gel, 5 % ethyl acetate in pet ether) to obtain the title compound.
Yield: 0.11 g (47.5 %). ¹H NMR (DMSO-d₆): δ 9.31 (bs, 2H), 7.44 (d, 1 H, J = 15.9 Hz), 7.00 (s, 1 H), 6.97 (d, 1 H), 6.73 (d, 1 H), 6.24 (d, 1 H, J = 15.9 Hz), 4.12 (q, 2H), 1.24 (t, 3H); MS (ES-): 207 (M-1).

### Example 4

### (E)-3-(3,4-dihydroxy-phenyl)-acrylic acid 2-nitro-ethyl ester (Compound 4)

Compound 1 (0.400 g, 2.22 mmol) was dissolved in tetrahydrofuran (30 mL) and cooled to 0 °C. To this, thionyl chloride (0.49 mL, 6.88 mmol) was added dropwise, maintaining the temperature at 0 °C, over a period of 30 mins. The reaction mixture was allowed to warm to room temperature (25 °C) and stirred for 2 hrs. The reaction mixture was concentrated to dryness under reduced pressure. The residue was dissolved in tetrahydrofuran (5 mL) and cooled to 0 °C. 2-Nitroethanol (1.59 mL, 22.20 mmol) was added dropwise and the reaction mixture allowed to warm to room temperature (25 °C) and stirred overnight (16 h). At the end of the reaction, the mixture was concentrated under reduced pressure. The residue was diluted with 10 % aqueous sodium bicarbonate solution to pH 7 and extracted with ethyl acetate (2 x 15 mL). The organic layer was washed with water (10 mL) and brine (10 mL), dried over anhydrous sodium sulfate and concentrated under reduced pressure. The residue was purified by flash chromatography on silica gel (1 % methanol in chloroform) to obtain the title compound.
Yield: 0.170 g (30.35 %). ¹H NMR (CDCl₃): δ 7.55 (d, 1 H, J = 15.9 Hz), 7.03 (d, 1 H), 6.94 (dd, 1 H), 6.76 (d, 1 H), 6.24 (d, 1 H, J = 15.9 Hz), 4.79 (m, 2H), 4.66 (m, 2H).

### Example 5

### 3-(3,4-Dihydroxy-phenyl)-acrylic acid n-propyl ester (Compound 5)

Compound 1 (0.25 g, 1.38 mmol) was dissolved in propan-1-ol (20 mL) and cooled to 0 °C. To this, thionyl chloride (0.12 mL, 1.65 mmol) was added dropwise maintaining the temperature at 0 °C for a period of 30 mins. The reaction mixture was allowed to warm to room temperature (25 °C), heated to 50 °C and maintained at that temperature overnight (16 h). The reaction mixture was concentrated under reduced pressure. The residue was diluted with 10 % sodium bicarbonate to pH 7 and extracted with ethyl acetate (2 x 10 mL). The organic layer was washed with water (5 mL) and brine (5 mL), dried over anhydrous sodium sulfate and concentrated under reduced pressure. The residue was purified by flash chromatography (silica gel, 5 % ethyl acetate in pet ether) to obtain the title compound.
Yield: 0.2 g (65.2 %). ¹H NMR (DMSO-d₆): δ 9.5 (bs, 2H), 7.40 (d, 1 H, J = 15.9 Hz), 7.02 (s, 1 H), 6.98 (d, 1 H), 6.73 (d, 1 H), 6.21 (d, 1 H, J = 15.9 Hz), 4.04 (t, 2H), 1.61 (m, 2H), 0.89 (t, 3H); MS (ES-): 221 (M-1).

### Example 6

### 3-(3,4-Dihydroxy-phenyl)-acrylic acid i-propyl ester (Compound 6)

Compound of Example 1 (0.3 g, 1.66 mmol) was dissolved in propan-2-ol (20 mL) and cooled to 0 °C. To this, thionyl chloride (0.18 mL, 2.5 mmol) was added dropwise maintaining the temperature at 0 °C for a period of 30 mins. The reaction mixture was allowed to warm to room temperature (25 °C), and then heated to 70 °C and maintained at that temperature overnight (16 h). The reaction mixture was concentrated under reduced pressure. The residue was diluted with 10 % sodium bicarbonate to pH 7 and extracted with ethyl acetate (2 x 10 mL). The organic layer was washed with water (5 mL) and brine (5 mL), dried over anhydrous sodium sulfate and concentrated under reduced pressure. The residue was purified by flash chromatography (silica gel, 3 % propan-2-ol in chloroform) to obtain the title compound.
Yield: 0.2 g (54.2 %). ¹H NMR (DMSO-d₆): δ 9.33 (bs, 2H), 7.42 (d, 1 H, J = 15.9 Hz), 7.00 (s, 1 H), 6.97 (d, 1 H), 6.73 (d, 1 H), 6.20 (d, 1 H, J = 15.9 Hz), 4.96 (m, 1 H), 1.21 (d, 6H); MS (ES-): 221 (M-1).

### Example 7

### 3-(3,4-Dihydroxy-phenyl)-acrylic acid butyl ester (Compound 7)

Compound 1 (0.1 g, 5.5 mmol) was dissolved in n-butanol (10 mL) and cooled to 0 °C. To this, thionyl chloride (0.04 mL, 8.3 mmol) was added dropwise maintaining the temperature at 0 °C for a period of 30 mins. The reaction mixture was allowed to warm to room temperature (25 °C) and stirred for 16 h. The reaction mixture was concentrated under reduced pressure. The residue was diluted with 10 % aqueous sodium bicarbonate solution to achieve pH 7 and extracted with ethyl acetate (2 x 25 mL). The organic layer was washed with water (10 mL) and brine (10 mL), dried over anhydrous sodium sulfate and concentrated under reduced pressure. The crude product obtained was purified by flash chromatography (silica gel, 5 % ethyl acetate in pet ether) to obtain the title compound.
Yield: 50.0 mg (38.4 %). ¹H NMR (CDCl₃): δ 7.56 (d, 1H, J = 15.9 Hz), 7.08 (s, 1 H), 7.01 (d, 1 H), 6.87 (d, 1 H), 6.25 (d, 1 H, J = 15.9 Hz), 5.86 (bs, 1 H), 5.80 (bs, 1 H), 4.19 (t, 2H), 1.70 (m, 2H), 1. 44 (m, 2H), 0.95 (t, 3H); MS (ES-) 235 (M-1).

### Example 8

### (E)-3-(3,4-dihydroxy-phenyl)-1-piperidin-1-yl-propenone (Compound 8)

Compound 1 (0.500 g, 2.77 mmol) was dissolved in tetrahydrofuran (30 mL) and cooled to 0 °C. To this, thionyl chloride (1 mL, 13.85 mmol) was added dropwise, maintaining the temperature at 0 °C, over a period of 30 mins. The reaction mixture was allowed to warm to room temperature (25 °C) and was stirred for 2 hrs. The reaction mixture was concentrated to dryness under reduced pressure. The residue was dissolved in tetrahydrofuran (10 mL) and cooled to 0 °C. Piperidine (1.1 mL, 11.08 mmol) was added dropwise and the reaction mixture allowed to warm to room temperature (25 °C) and stirred overnight (16 h). At the end of the reaction, the mixture was concentrated under reduced pressure. The residue was diluted with 10 % aqueous sodium bicarbonate solution to pH 7 and extracted with ethyl acetate (2 x 25 mL). The organic layer was washed with water (10 mL) and brine (10 mL), dried over anhydrous sodium sulfate and concentrated under reduced pressure. The residue was purified by flash chromatography on silica gel (2 % methanol in chloroform) to obtain the title compound.
Yield: 0.350 g (51.02 %). ¹H NMR (DMSO-d₆): δ 7.27 (d, 1 H, J = 15.3 Hz), 7.04 (d, 1 H), 6.95 (dd, 1H,), 6.89 (d, 1 H, J = 15.3Hz), 6.71 (d, 1H,), 3.53 (m, 4H), 1.57 (m, 6H); MS (ES-): 246.11.

### Example 9

3-(3,4-Dihydroxy-phenyl)-1-(4-ethyl-piperazin-1-yl)-propenone (Compound 9) Compound 1 (0.3 g, 1.66 mmol) was dissolved in tetrahydrofuran (5 mL) and cooled to 0 °C. To this, thionyl chloride (0.36 mL, 5.1 mmol) was added dropwise maintaining the temperature at 0 °C for a period of 30 mins. The reaction mixture was allowed to warm to room temperature (25 °C) and stirred for 2 hrs. The reaction mixture was concentrate to dryness under reduced pressure. The residue was dissolved in tetrahydrofuran (5 mL) and cooled to 0 °C. 1-Ethyl-piperazine (1.1 mL, 8.62 mmol) was added dropwise and the reaction mixture allowed to warm to room temperature (25 °C) and stirred overnight (16 h). The reaction mixture was concentrated under reduced pressure. The residue was diluted with 10 % aqueous sodium bicarbonate solution to pH 7 and extracted with ethyl acetate (2 x 25 mL). The organic layer was washed with water (10 mL) and brine (10 mL), dried over anhydrous sodium sulfate and concentrated under reduced pressure. The residue was purified by flash chromatography (silica gel, 5 % methanol in chloroform) to obtain the title compound.
Yield: 180 mg (39.2 %). ¹H NMR (DMSO-d₆): δ 9.43 (s, 1 H), 8.97 (s, 1 H), 7.45 (d, 1 H, J = 15.9 Hz), 7.05 (d, 1 H), 6.96 (dd, 1 H), 6.89 (d, 1H, J = 15.9 Hz), 6.71 (d, 1 H), 3.56 (m, 4H), 2.32 (m, 6H), 0.99 (t, 3H); MS (ES+): 277 (M+1).

### Preparation of hydrochloride salt:

### 4-[3-(3,4-Dihydroxy-phenyl)-acryloyl]-1-ethyl-piperazin-1-ium chloride

Compound 9 (2.0 g, 7.29 mmol) was dissolved in 10 mL methanol. The solution was cooled to 0 - 5 °C, 5 % ethereal hydrochloric acid solution (25 mL) was added and stirred for 10 mins. The reaction mixture was allowed to warm to room temperature (25 °C) and stirred for 1 hr. The reaction mixture was concentrated under reduced pressure and the resulting solid was dried completely to obtain the desired product. Yield: 2.1 g (93.3 %). ¹H NMR (DMSO-d₆): δ10.88 (s, 1 H), 7.35 (d, 1 H, J = 15.3 Hz), 7.10 (d, 1 H, J = 1.5 Hz), 6.99 (d, 1H, J = 1.5 Hz), 6.97 (d, 1 H, J = 15.3 Hz), 6.74 (d, 1 H, J = 8.4 Hz), 4.48 (m, 2H), 3.46 (m, 2H), 3.11 (m, 4H), 2.91 (m, 2H), 1.23 (t, 3H).

### Example 10

### 1-(4-Benzyl-piperazin-1-yl) 3-(3,4-dihydroxy-phenyl)-propenone (Compound 10)

Compound 1 (0.3 g, 1.66 mmol) was dissolved in tetrahydrofuran (5 mL) and cooled to 0 °C. To this, thionyl chloride (0.6 mL, 8.5 mmol) was added dropwise maintaining the temperature at 0 °C for a period of 30 mins. The reaction mixture was allowed to warm to room temperature (25 °C) and stirred for 2 hrs. The reaction mixture was concentrated to dryness under reduced pressure. The residue was dissolved in tetrahydrofuran (5 mL) and cooled to 0 °C. 1-Benzyl-piperazine (0.9 mL, 5.2 mmol) was added dropwise and the reaction mixture allowed to warm to room temperature (25 °C) and stirred overnight (16 h). The reaction mixture was concentrated under reduced pressure. The residue was diluted with 10 % aqueous sodium bicarbonate solution to pH 7 and extracted with ethyl acetate (2 x 25 mL). The organic layer was washed with water (10 mL) and brine (10 mL), dried over anhydrous sodium sulfate and concentrated under reduced pressure. The residue was purified by flash chromatography (silica gel, 5 % methanol in chloroform) to obtain the title compound. Yield: 160 mg (28.4 %). ¹H NMR (CD₃OD): δ 7.44 (d, 1 H, J = 15.3 Hz), 7.32 (m, 5H), 7.03 (d, 1H), 6.96 (dd, 1 H), 6.85 (d, 1 H, J = 15.3 Hz), 6.75 (d, 1H), 3.71 (bs, 4H), 3.56 (s, 2H), 2.49 (bs, 4H); MS (ES+): 339 (M+1).

### Preparation of hydrochloride salt:

### 4-[3-(3,4-Dihydroxy-phenyl)-acryloyl]-1-benzyl-piperazin-1-ium chloride

Compound 10 (0.5 g, 1.49 mmol) was dissolved in 15 mL methanol. The solution was cooled to 0 - 5 °C, 5 % ethereal hydrochloric acid solution (25 mL) was added and stirred for 10 mins. The reaction mixture was allowed to warm to room temperature (25 °C) and stirred for 1 hr. The reaction mixture was concentrated under reduced pressure and the resulting solid was dried completely to obtain the desired product.
Yield: 0.52 g (94.54 %). ¹H NMR (DMSO-d₆): δ 9.56 (s, 1 H), 7.56 (m, 2H), 7.32 (d, 1 H J = 15.3 Hz), 9.06 (d, 1 H), 6.95 (m, 1 H), 6.89 (d, 1 H, J = 15.3 Hz), 6.72 (d, 1 H), 4.43 (m, 2H), 4.27 (m, 2H), 3.26 (m, 3H), 2.89 (m, 3H).

### Example 11

### (E)-3-{4-[3-(3,4-dihydroxy-phenyl)-acryloyl]-piperazin-1-yl} propionitrile (Compound 11)

Compound 1 (0.400 g, 2.22 mmol) was dissolved in tetrahydrofuran (30 mL) and cooled to 0 °C. To this, oxalyl chloride (0.57 mL, 6.66 mmol) was added drop wise, maintaining the temperature at 0 °C, over a period of 30 mins. The reaction mixture was allowed to warm to room temperature (25 °C) and stirred for 2 hrs. The reaction mixture was concentrated to dryness under reduced pressure. The residue was dissolved in tetrahydrofuran (15 mL) and cooled to 0 °C under nitrogen atmosphere. This was added dropwise to a solution of 3-piperazin-1-yl-propionitrile (0.37 g, 2.66 mmol) and triethylamine (0.67 ml, 48.8 mmol) in tetrahydrofuran maintained at 0 °C. The reaction mixture was allowed to warm to room temperature (25 °C) and stirred overnight (16 h). At the end of the reaction, the mixture was concentrated under reduced pressure and purified by flash chromatography on silica gel (1 % methanol in chloroform) to obtain the title compound.
Yield: 0.130 g (19.43 %). ¹H NMR (CD₃OD): δ 7.45 (d, 1 H, J = 15 Hz), 7.04 (d, 1 H), 6.97 (dd, 1 H), 6.86 (d, 1 H, J = 15.3 Hz), 6.76 (d, 1 H), 3.75 (m, 4H), 2.71 (m, 4H), 2.66 (m, 4H); MS (ES+): 302.14.

### Example 12

### (E)-3-(3,4-dihydroxy-phenyl)-1-[4-(1-methyl-piperidin-4-yl) piperazin-1-yl]- propenone (Compound 12)

Compound 1 (0.500 g, 2.77 mmol) was dissolved in tetrahydrofuran (30 mL) and cooled to 0°C. To this, oxalyl chloride (0.52 mL, 6.09 mmol) was added drop wise, maintaining the temperature at 0 °C, over a period of 30 mins. The reaction mixture was allowed to warm to room temperature (25 °C) and stirred for 3 hrs. The reaction mixture was concentrated to dryness under reduced pressure. The residue was dissolved in tetrahydrofuran (10 mL) and cooled to 0 °C under nitrogen atmosphere. This was added dropwise to a solution of 1-(1-methylpiperidin-4-yl) piperazine (0.45 g, 2.49 mmol) and triethylamine (0.84 mL, 6.09 mmol) in tetrahydrofuran, maintained 0 °C. The reaction mixture was allowed to warm to room temperature (25 °C) and stirred (16 h). At the end of the reaction, the mixture was concentrated under reduced pressure and the residue purified by flash chromatography on silica gel (1 % methanol in chloroform) to obtain the title compound.
Yield: 0.170 g, (17.74 %). ¹H NMR (CD₃OD): δ 7.47 (d, 1 H, J = 15.3 Hz), 7.06 (d, 1 H), 6.98 (dd, 1 H), 6.87 (d, 1 H, J = 15.3 Hz), 6.79 (d, 1 H), 3.76 (bs, 4H), 3.18 (m, 2H), 2.63 (bs, 4H), 2.51 (s, 3H), 2.45 (m, 3H), 1.96 (m, 2H), 1.69 (m, 2H); MS (ES+): 346.21.

### Example 13

(E)-3-(3,4-dihydroxy-phenyl)-1-(4-phenylpiperazin-1-yl)-propenone (Compound 13) Compound 1 (0.500 g, 2.77 mmol) was dissolved in tetrahydrofuran (30 mL) and cooled to 0°C. to this, oxalyl chloride (0.71 mL, 8.31 mmol) was added drop wise, maintaining the temperature at 0 °C, over a period of 30 mins. The reaction mixture was allowed to warm to room temperature (25 °C) and stirred for 3 hrs. The reaction mixture was concentrated to dryness under reduced pressure. The residue was dissolved in tetrahydrofuran (10 mL) and cooled to 0 °C under nitrogen atmosphere. This was added dropwise to a solution of N-phenyl-piperazine (0.54 mL, 3.60 mmol) and triethylamine (0.84 mL, 6.09 mmol) in tetrahydrofuran, maintained at 0 °C. The reaction mixture was allowed to warm to room temperature (25 °C) and stirred overnight (16 h). At the end of the reaction, the mixture was concentrated under reduced pressure and the residue purified by flash chromatography on silica gel (1 % methanol in chloroform) to obtain the title compound.
Yield: 0.100 mg (11.11 %). ¹H NMR (CD₃OD) δ 7.47 (d, 1 H, J = 15.3 Hz), 7.23 (t, 2H), 7.05 (d, 1 H), 6.98(d, 2H), 6.91 (1H, J = 15.3 Hz), 6.84 (m, 1 H), 6.77 (d, 1 H), 3.85 (bs, 4H), 3.19 (bs, 4H); MS (ES-): 323.12.

### Example 14

(E)-1-(4-acetyl-piperazin-1-yl)-3-(3,4-dihydroxy-phenyl)-propenone (Compound 14) Compound 1 (0.500 g, 2.77 mmol) was dissolved in tetrahydrofuran (30 mL) and cooled to 0 °C. To this, oxalyl chloride (0.52 mL, 6.09 mmol) was added dropwise, maintaining the temperature at 0 °C, over a period of 30 mins. The reaction mixture was allowed to warm to room temperature (25 °C) and stirred for 3 hrs. The reaction mixture was concentrated to dryness under reduced pressure. The residue was dissolved in tetrahydrofuran (10 mL) and cooled to 0 °C under nitrogen atmosphere. This was added dropwise to a solution of N-acetyl-piperazine (0.35 g, 2.77 mmol) and triethylamine (0.84 mL, 6.09 mmol) in tetrahydrofuran, maintained at 0°C. The reaction mixture allowed to warm to room temperature (25 °C) and stirred overnight (16 h). At the end of the reaction, the mixture was concentrated under reduced pressure and the residue purified by flash chromatography on silica gel (1 % methanol in chloroform) to obtain the title compound.
Yield: 0.3 g (37.26 %). ¹H NMR (DMSO-d₆): δ 8.93 (bs, 1 H), 7.34 (d, 1 H, J = 15.3 Hz), 6.99 (dd, 1 H), 7.09 (d, 1H, J = 15Hz), 6.75 (d, 1 H), 3.85 (m, 8H), 2.03 (s, 3H);
MS (ES+): 291.15 (M+1).

### Example 15

### (E)-3-(3,4-dihydroxy-phenyl)-1-(4-phenethyl-piperazin-1-yl)-propenone

### (Compound 15)

Compound 1 (0.500 g, 2.77 mmol) was dissolved in tetrahydrofuran (30 mL) and cooled to 0°C. To this, oxalyl chloride (0.35 mL, 4.15 mmol) was added drop wise, maintaining the temperature at 0 °C, over a period of 30 mins. The reaction mixture was allowed to warm to room temperature (25 °C) and stirred for 3 hrs. The reaction mixture was concentrated to dryness under reduced pressure. The residue was dissolved in tetrahydrofuran (10 mL) and cooled to 0 °C under nitrogen atmosphere. This was added dropwise to a solution of 1-phenylethyl-piperazine (0.52 g, 2.77 mmol) and triethylamine (0.84 mL, 6.09 mmol) in tetrahydrofuran, maintained at 0°C. The reaction mixture was allowed to warm to room temperature (25 °C) and stirred overnight (16 h). At the end of the reaction, the mixture was concentrated under reduced pressure and the residue purified by flash chromatography on silica gel (1 % methanol in chloroform) to obtain the title compound.
Yield: 0.150 g (15.33 %). MS (ES+): 353.24.

### Example 16

### (E)-3-(3,4-dihydroxy-phenyl)-1-morpholin-4-yl-propenone (Compound 16)

Compound of Example 1 (0.4 g, 2.22 mmol) was dissolved in tetrahydrofuran (30 mL) and cooled to 0 °C. To this, thionyl chloride (0.51 mL, 7.10 mmol) was added dropwise, maintaining the temperature at 0 °C, over a period of 30 mins. The reaction mixture was allowed to warm to room temperature (25 °C) and stirred for 2 hrs. The reaction mixture was concentrated to dryness under reduced pressure. The residue was dissolved in tetrahydrofuran (10 mL) and cooled to 0 °C. Morpholine (0.77 mL, 8.88 mmol) was added dropwise and the reaction mixture allowed to warm to room temperature (25 °C) and stirred overnight (16 h). At the end of the reaction, the mixture was concentrated under reduced pressure and the residue purified by flash chromatography on silica gel (1 % methanol in chloroform) to obtain the title compound.
Yield: 0.200 g (28.94 %). ¹H NMR (DMSO-d₆): δ 7.31 (d, 1 H, J = 15 Hz), 7.06 (bs, 1 H), 6.96 (d, 1 H), 6.89 (d, 1 H, J = 15.3 Hz), 6.71 (d, 1 H), 3.60 (m, 8H).

### Example 17

### 3-(3,4-dihydroxy-phenyl)-N-(3-dimethylamino-propyl)-acrylamide (Compound 17)

A mixture of compound 1 (0.2 g, 1.11 mmol), N,N-dimethyl-propane-1,3-diamine (0.13 mL, 1.11 mmol) and HOBt (0.2 g, 1.32 mmol) was dissolved in N,N-dimethylformamide (5 mL). Triethylamine (0.4 mL, 3.3 mmol) was added to this solution at 0 °C. After 10 min, EDC (0.25 g, 1.3 mmol) was added at 0 °C and the reaction mixture was stirred at room temperature (25 °C) for 24 h. The mixture was concentrated under, reduced pressure and the residue was diluted with ethyl acetate (25 mL). The organic layer was washed with water (10 mL), brine (10 mL) and dried over anhydrous sodium sulfate and concentrated under reduced pressure. The crude product obtained was purified by flash chromatography (silica gel, 0.3 % methanol in chloroform) to obtain the title compound.
Yield: 190 mg (64.7 %). MS (ES-): 263 (M-1).

### Example 18

### (E)-3-(3,4-dihydroxy-phenyl)-N-isopropyl-acrylamide (Compound 18)

Compound 1 (0.500 g, 2.77 mmol) was dissolved in tetrahydrofuran (30 mL) and cooled to 0°C. To this, oxalyl chloride (0.71 mL, 8.31 mmol) was added dropwise, maintaining the temperature at 0 °C, over a period of 30 mins. The reaction mixture was allowed to warm to room temperature (25 °C) and stirred for 3 hrs. The reaction mixture was concentrated to dryness under reduced pressure. The residue was dissolved in tetrahydrofuran (10 mL) and cooled to 0 °C under nitrogen atmosphere. This was added dropwise to a solution of isopropyl amine (0.30 mL, 3.60 mmol) and triethylamine (0.84 mL, 6.09 mmol) in tetrahydrofuran, maintained at 0°C. The reaction mixture was allowed to warm to room temperature (25 °C) and stirred overnight (16 h). At the end of the reaction, the mixture was concentrated under reduced pressure, and the residue purified by flash chromatography on silica gel (1 % methanol in chloroform) to obtain title compound.
Yield: 0.150 g (24.42%). ¹H NMR (CD₃OD): δ 7.36 (d, 1 H, J =15.6 Hz), 6.98 (d, 1 H), 6.88(dd, 1 H), 6.74 (d, 1 H), 6.32 (d, 1 H, J = 15.6Hz), 4.06 (m, 1 H), 1.17 (d, 6H).

### Example 19

### 1-(4-Benzyl-piperidin-1-yl)-3-(3,4-dihydroxy-phenyl)-propenone

Compound 1 (5 g, 27.7 mmol) was dissolved in tetrahydrofuran (60 mL) and cooled to 0 °C. To this, oxalyl chloride (5.26 mL, 61.0 mmol) was added drop wise maintaining the temperature at 0 °C for a period of 30 mins. The reaction mixture was allowed to warm to room temperature (25 °C) and stirred for 3 hrs. The reaction mixture was concentrated to dryness under reduced pressure. The residue was dissolved in tetrahydrofuran (20 mL) and cooled to 0 °C under nitrogen atmosphere. This was added dropwise to a solution of 4-benzylpiperidine (5.35 g, 30.5 mmol) and triethylamine (5.52 mL, 41.6 mmol) in tetrahydrofuran maintained at 0 °C. The reaction mixture allowed to warm to room temperature (25 °C) and stirred overnight (16 h). The reaction mixture was concentrated under reduced pressure and purified by flash chromatography (silica gel, 1 % methanol in chloroform) to obtain the title compound. Yield: 2.2 g (23.50 %). ¹H NMR (CD₃OD): δ7.41 (d, 1H, J=15.3 Hz), 7.25 (m, 2H), 7.15 (m, 3H), 7.02 (d, 1 H), 6.95 (dd, 1 H), 6.85 (d, 1 H, J= 15.3 Hz), 6.76 (d, 1 H), 4.57 (m, 1 H), 4.20 (m, 1 H), 3.08 (m, 1 H), 2.67 (m, 1 H), 2.56 (d, 2H), 1.85 (m, 1 H), 1.73 (m, 2H), 1.18(m, 2H); MS (ES-): 336.1 (M-1).

The efficacy of the compounds of the present invention in inhibiting the Gleevec sensitive cell line K-562, Ba/F3 Bcr-Abl/WT and Gleevec resistant cell lines K-562-R and 320cl^{Bcr-Abl T315I} , Ba/F3 Bcr-Abl/T315l, Ba/F3 Bcr-Abl/E255K, Ba/F3 Bcr-Abl/H396P, Ba/F3 Bcr-Abl/M351T, Ba/F3 Bcr-Abl/F359V, Ba/F3 Bcr-Abl/E255V, Ba/F3 Bcr-Abl/F317L, Ba/F3 Bcr-Abl/H396R, Ba/F3 Bcr-Abl/M244V, Ba/F3 Bcr-Abl/Q252H, Ba/F3 Bcr-Abl/Y253F and Ba/F3 Bcr-Abl/Y253H can be determined by number of pharmacological assays described below. The exemplified pharmacological assays, which follow, have been carried out with imatinib mesylate, and compounds of the present invention or their salts (referred to herein as test compounds).

The following abbreviations are used herein:
ATCC : American Type Culture Collection
OHSU : Oregon Health and Science University, Oregon, USA
NPRC : Nicholas Piramal Research Centre, Mumbai, India
FBS : Fetal Bovine Serum
IMDM : Iscove's Modified Dulbecco's Medium
RPMI : Roswell Park Memorial Institute
SDS-PAGE : Sodium dodecyl sulphate polyacrylamide gel electrophoresis
PBS : Phosphate buffered saline
EDTA : Ethylene diamine tetra acetate
NP-40 : Nonidet P-40
HEPES : (N-[2-Hydroxy ethyl] piperazine-N'-[2-ethane sulphonic acid])
DTT : Dithiothreitol
EGTA : Ethylene glycol bis(2-aminoethyl ether)-N,N,N'N'- tetraacetic acid
IL3 : Interleukin 3

### Example 20

### Cell proliferation assay

³H-Thymidine uptake assay: ³H-Thymidine-incorporation is directly proportional to the number of dividing cells in culture.

### Protocol:

Cell lines were procured from different sources (Table1) and were maintained under optimum conditions of growth as suggested by the supplier. Exponentially growing cultures were subjected to different concentrations of test compounds and standard as given below.

**Table 1: Description of various hematopoietic cell lines**

| **Sr. No.** | **Cell line** | **Description of the cell line** | **Source** | **Ph status** | **Medium of propagation (FBS%)** |
|---|---|---|---|---|---|
| 1 | K-562 | Human Bcr-Abl Leukemia cell line | ATCC | +ve | IMDM (10%) |
| 2 | K-562-R | Human Bcr-Abl Leukemia cell line resistant to Gleevec | NPRC | +ve | RPMI-1640 (10%) |
| 3 | 32DCl3 Bcr-Abl | Mouse fibroblast with T315I mutated Bcr-Abl | - | +ve | IMDM (10%) |

### Method:

Cells were seeded at a density of 3 x 10³ to 5 x 10³ per well (in 0.180 mL) in transparent 96 well tissue culture plate (NUNC, USA) and were allowed to incubate at 37 °C in 5% carbon dioxide incubator for 2-6 h. Test compounds were diluted in medium (RPMl1640 with 10% FBS) at various concentrations, and 0.02 mL of 10x stocks were added to each well in triplicate. Plates were incubated at 37 °C in 5% carbon dioxide incubator for 72 h, with an intermittent microscopic observation every 24 h.

After 72 h incubation, the plate was centrifuged at 1000 rpm for 10 mins in a plate centrifuge. Supernatant was carefully aspirated and ³H-Thymidine was added to all wells at a concentration of 0.5 µCi/well in 0.1 mL complete medium. Plates were further incubated at 37°C in 5 % carbon dioxide incubator for 6-14 h.

At the end of incubation period, cells from 96 well plates were harvested with the help of a cell harvester (Packard, USA) on 96 well glass-filter plate (Cat # 6005177, Unifilter-96, GF/B, Packard, USA). The filter plate was dried completely at 60 °C for 1 h, or overnight (16 h) at room temperature (25 °C). After drying, bottom of the plate was blocked with seal and 0.05 mL/well of scintillant fluid (Microscint-O, Packard) was added. The plate was sealed from the top, read on scintillation counter (TopCount, Packard), and percent inhibition and IC₅₀ in comparison with control values was calculated.

**Table 2: Antiproliferative activity of compound 2 with various cell lines**

| **No.** | **Compound** | **IC₅₀ µM** | | |
|---|---|---|---|---|
| | | **K-562** | **K-562-R** | **32D_{cl}^{Bcr-Abl T315I}** |
| 1 | Imitanib mesylate | 0.21 ± 0.05 | 1.0 | >3.0 |
| 2 | Compound 2 | 13.6 ± 2.3 | 23.0 | 35.0 |

Conclusion: Compound 2 of the present invention inhibited proliferation of the Gleevec resistant cells K-562, K-562-R and 32Dcl^{Bcr-Abl T315I}.

### Example 21

Determination of specificity of the test compounds for imatinib mesylate resistant cell lines using *in-vitro* antiproliferative activity.

Several imatinib mesylate-resistant cell lines were procured from Dr. Brian Druker's laboratory, Howard Hughes Medical Institute, Oregon Health and Science University (OHSU) Cancer Institute, Portland, Oregon, USA, the details of which are provided in the Table 3. These cell lines were maintained under optimum conditions of growth as suggested by the supplier.

**Table 3: Description of various imatinib mesylate sensitive and imatinib mesylate resistant cell lines**

| **Sr. No** | **Cell line** | **Source** | **Ph status** | **Medium of propagation (FBS%)** |
|---|---|---|---|---|
| 1 | Ba/F3 Bcr-Abl/ WT | OHSU | + ve wild type | RPMI-1640 (10%) |
| 2 | Ba/F3 Bcr-Abl/T315I | OHSU | + ve mutated | RPMI-1640 (10%) |
| 3 | Ba/F3 Bcr-Abl/E255K | OHSU | + ve mutated | RPMI-1640 (10%) |
| 4 | Ba/F3 Bcr-Abl/H396P | OHSU | + ve mutated | RPMI-1640 (10%) |
| 5 | Ba/F3 Bcr-Abl/M351T | OHSU | + ve mutated | RPMI-1640 (10%) |
| 6 | Ba/F3 Bcr-Abl/F359V | OHSU | + ve mutated | RPMI-1640 (10%) |
| 7 | Ba/F3 Bcr-Abl/E255V | OHSU | + ve wild type | RPMI-1640 (10%) |
| 8 | Ba/F3 Bcr-Abl/F317L | OHSU | + ve mutated | RPMI-1640 (10%) |
| 9 | Ba/F3 Bcr-Abl/H396R | OHSU | + ve mutated | RPMI-1640 (10%) |
| 10 | Ba/F3 Bcr-Abl/M244V | OHSU | + ve mutated | RPMI-1640 (10%) |
| 11 | Ba/F3 Bcr-Abl/Q252H | OHSU | + ve mutated | RPMI-1640 (10%) |
| 12 | Ba/F3 Bcr-Abl/Y253F | OHSU | + ve mutated | RPMI-1640 (10%) |
| 13 | Ba/F3 Bcr-Abl/Y253H | OHSU | + ve mutated | RPMI-1640 (10%) |
| 14 | Ba/F3 * | OHSU | - ve wild type | IMDM (10%)+IL3** |

| | | | | |
|---|---|---|---|---|
| * Ba/F3- cells containing vector only (Ba/F3-pSR α) ** IL3 as growth factor | | | | |

The cell lines described in Table 3 were used to test the antiproliferative activity of test compounds. The activity of the test compounds as an antiproliferative agent was compared with imatinib mesylate in imatinib mesylate-resistant cell lines using CCK-8 assay.

### Cell proliferation and cytotoxicity CCK-8 assay:

Cell Counting Kit-8 (CCK-8) allows very convenient assay by utilizing Dojindo's highly water-soluble tetrazolium salt. CCK-8, being nonradioactive, allows sensitive colorimetric assay for the determination of number of viable cells in cell proliferation and cytotoxicity assays. The amount of the formazan dye generated by dehydrogenases in cells is directly proportional to the number of living cells. Thus, the CCK-8 assay can also be substituted for the H³-Thymidine incorporation assay.

### Compound preparation

Imatinib mesylate was purchased from Natco Pharma, India. For the test compounds and standard imatinib mesylate, 10 mM stock was prepared in DMSO.

### Method

Cells were seeded at a density of ~ 5 x 10³ per well (0.09 mL) in a transparent 96-well tissue culture plate (NUNC, USA) and allowed to incubate at 37 °C, 5% CO₂ incubator for 2-6 h. Different concentrations of test compounds and a standard imatinib mesylate were added to each well in triplicate. Plates were further incubated at 37 °C, 5% CO₂ incubator for 72 h. 10 µl of the CCK-8 solution was added to each well and plate was incubated for 1-4 h in the incubator. Measured the absorbance at 450 nm using a microplate reader.

**A**. The percent inhibition and IC₅₀ were calculated in comparison with control values. The results are provided in the following Table 4 and 5.

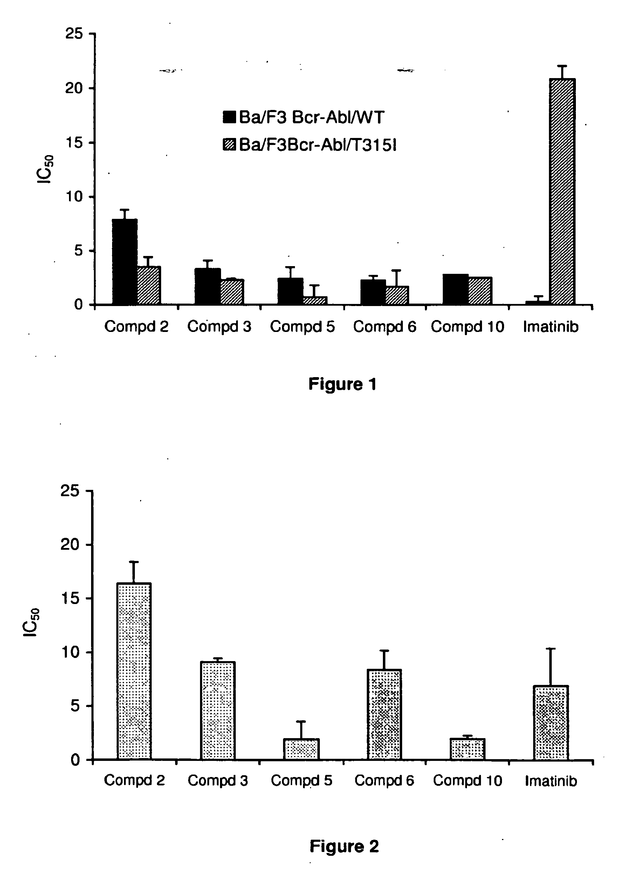

**B**. Anti-proliferative activity of the test compounds, expressed as IC₅₀ values in µM, in imatinib mesylate sensitive (Ba/F3 Bcr-Abl/WT) and resistant (Ba/F3 Bcr-Abl/T315I) as seen in the clinic, is represented by graph as shown in Figure 1. Anti-proliferative activity of compounds, expressed as mean IC₅₀ values in µM for five cell lines, in imatinib mesylate resistant low frequency mutations (Ba/F3 Bcr-Abl/E255K, Ba/F3 Bcr-Abl/H396P, Ba/F3 Bcr-Abl/E255V, Ba/F3 Bcr-Abl/M244V and Ba/F3 Bcr-Abl/Q252H) as seen in the clinic is represented by graph as shown in Figure 2.

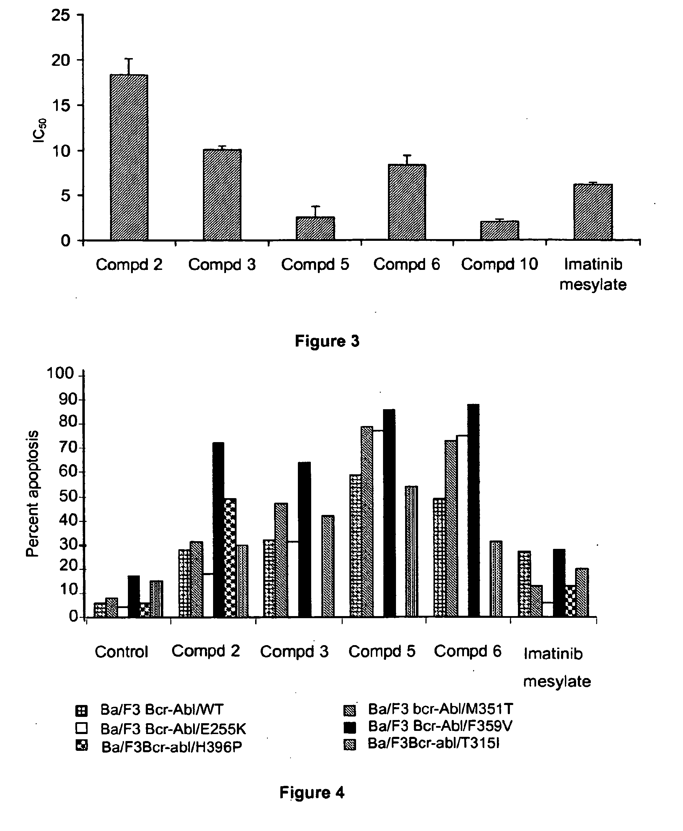

Anti-proliferative activity of compounds, expressed as mean IC₅₀ values in µM for seven cell lines, in imatinib mesylate resistant high frequency mutations (Ba/F3 Bcr-Abl/T315l, Ba/F3 Bcr-Abl/M351T, Ba/F3 Bcr-Abl/F359V, Ba/F3 Bcr-Abl/F317L, Ba/F3 Bcr-Abl/H396R, Ba/F3 Bcr-Abl/Y253F and Ba/F3 Bcr-Abl/Y253H) as seen in the clinic is represented by graph as shown in Figure 3.

**Table 4: Inhibitory concentrations (IC₅₀) for compounds of the present invention in imatinib mesylate sensitive and resistant cell lines.**

| **Sr. No** | **Compounds** | **Cell lines (IC₅₀) µM** | | |
|---|---|---|---|---|
| | | **Imatinib mesylate Sensitive** | | **Imatinib mesylate Resistant** |
| | | **K562** | **Ba/F3 Bcr-Abl/WT** | **Ba/F3 Bcr-Abl/T315I** |
| 1 | Compound 1 (reference) | >100 | >100 | >100 |
| 2 | Compound 2 | 13.3 ± 2.1 | 7.9 ± 2.1 | 3.8 ± 1.9 |
| 3 | Compound 3 | 11.8 ± 8.8 | 3.3 ± 0.9 | 2.3 ± 0.9 |
| 4 | Compound 5 | 13.5 ± 2.1 | 2.4 ± 0.8 | 0.7 ± 0.1 |
| 5 | Compound 6 | 16.5 ± 2.1 | 2.3 ± 1.1 | 1.7 ± 1.1 |
| 6 | Compound 9 | 59.5 | 40.6 ± 16.4 | 43.1 ± 27.9 |
| 7 | Compound 10 | 18.0 | 2.8 ± 0.4 | 2.5 ± 1.5 |
| 8 | Compound 12 | >30 | >30 | >30 |
| 9 | Compound 13 | 33.5 | 27.0 ± 8.5 | 26.6 ± 6.5 |
| 10 | Compound 18 | 32.1 | 19.4 ± 8.8 | 9.5 ± 4.1 |
| 11 | Compound 19 | 5.7 | 4.2 ± 1.3 | 2.7 ± 0.5 |
| 12 | Imatinib mesylate | 0.5 ± 0.2 | 0.3 ± 0.1 | 20.8 ± 6.1 |

**Table 5: Cytotoxic inhibitory activity of compounds of the present invention in imatinib mesylate resistant cell lines**

| **Sr. No** | **Cell-lines** | **Cytotoxic Inhibitory Activity (IC₅₀) µM** | | | | | | | |
|---|---|---|---|---|---|---|---|---|---|
| | | **I** | **II** | **III** | **IV** | **V** | **VI** | **VII** | **VIII** |
| 1 | Ba/F3 Bcr-Abl/E255K | >100 | 15.0 | 6.7 | 2.9 | 5.1 | 2.4 | 1.5 | 8.7 |
| 2 | Ba/F3 Bcr-Abl/E255V | >100 | 18.9 | 10.2 | 3.4 | 9.9 | 2.1 | 0.8 | 8.4 |
| 3 | Ba/F3 Bcr-Abl/F317L | >100 | 23.5 | 12.3 | 1.6 | 11.3 | 1.2 | 1.1 | 2.0 |
| 4 | Ba/F3 Bcr-Abl/F359V | >100 | 18.4 | 13.4 | 2.5 | 14.6 | 2.6 | 2.4 | 2.1 |
| 5 | Ba/F3 Bcr-Abl/H396P | >100 | 26.2 | 16.6 | 5.6 | 10.8 | 3.7 | 2.8 | 4.3 |
| 6 | Ba/F3 Bcr-Abl/H396R | >100 | 18.8 | 8.6 | 1.1 | 7.0 | 1.0 | 1.4 | 2.1 |
| 7 | Ba/F3 Bcr-Abl/M244V | >100 | 30.1 | 15.2 | 3.0 | 9.8 | 1.3 | 1.0 | 1.8 |
| 8 | Ba/F3 Bcr-Abl/M351T | >100 | 16.1 | 8.7 | 2.2 | 5.1 | 2.2 | 2.1 | 2.1 |
| 9 | Ba/F3 Bcr-Abl/Q252H | >100 | 15.4 | 8.4 | 1.7 | 5.9 | 1.5 | 1.1 | 2.0 |
| 10 | Ba/F3 Bcr-Abl/Y253F | >100 | 17.0 | 6.4 | 1.7 | 5.2 | 1.1 | 1.0 | 3.9 |
| 11 | Ba/F3 Bcr-Abl/Y253H | >100 | 14.5 | 13.2 | 3.3 | 13.3 | 2.5 | 1.0 | 9.2 |
| 12 | Ba/F3 Bcr-Abl/T315I | >100 | 6.3 | 1.1 | 0.6 | 2.0 | 3.1 | 2.1 | 27.0 |

| | | | | | | | | | |
|---|---|---|---|---|---|---|---|---|---|
| **I**- compound **1**, **II**- compound 2, **III**- compounds 3, **IV**- compound 5, **V-** compound 6, **VI-** compound 10, **VII**- compound 19, **VIII**- Imatinib mesylate | | | | | | | | | |

**Conclusion:** It is evident from the results that compounds of the present invention exhibited significant inhibitory activity against Bcr-Abl mutated imatinib mesylate resistant cells.

### Example 22

### Bcr-abl kinase assay

### Objective:

To study inhibition of autophosphorylation of Bcr-abl. Study was carried out by immunoprecipitation of p210^{bcr-abl} tyrosine kinase from K-562 cell lysate, followed by kinase enzyme assay. The reaction mixture was subjected to SDS-PAGE followed by autoradiography.

### Introduction:

Immunoprecipitation is a technique that permits the purification of specific proteins using specific antibodies, from the crude cell lysate. This primary antibody is either already bound to agarose or can be bound to the protein-A-Sepharose beads during the procedure in order to physically separate the antibody-antigen complex from the remaining sample.

### Method:

To check for the inhibition of autophosphorylation of p210 ^{bcr-abl} tyrosine kinase by compounds of the present invention, K-562 cells which express wild type Bcr-abl were used. 2 × 10⁶ K-562 cells were treated with compounds at IC₅₀ and 3 × IC₅₀ concentrations (based on antiproliferative activity data with K-562, as described in Example 20) and incubated for 24 h in humidified 5 % CO₂ incubator. Cells were washed with PBS solution and lysed using lysis buffer (CelLytic M Cell Lysis reagent, Sigma Aldrich, USA) for 2-3 h in cold conditions. Protein was estimated by Bradford method (Bradford reagent, Sigma Aldrich, USA). For immunoprecipitation equal amount of protein is taken from all samples. A p210^{bcr-abl} protein was immunoprecipitated using polyclonal anti-c-abl antibody (Santa Cruz Biotechnologies, USA) followed by incubation at 4 °C overnight with Protein A-Sepharose. Immune complex was isolated and washed three times in NET-N buffer (20 mM Tris-HCl pH 7.5, 100 mM NaCl, 1 mM EDTA and 0.5 % NP40) followed by resuspension in kinase buffer (50 mM HEPES pH 7.5, 1 mM DTT, 2.5 EGTA, 10 mM β-glycerophosphate, 1 mM NaF and 10 mM MgCl₂) solution.

Kinase reaction was performed using 0.5 µCi (γ³²P)-adenosine triphosphate per reaction and incubated for 30 minutes at room temperature (25°C). Kinase reaction is stopped by addition of SDS sample buffer and after heating at 95 °C for 5 minutes. Autophosphorylation reaction products are resolved on SDS-PAGE and detected by autoradiography.

**Results:** Immunoprecipitation followed by SDS-PAGE and autoradiography indicated reduction in Bcr-abl auto phosphorylation as seen by decreased band density. Compound 2 is found to be a potent Bcr-abl tyrosine kinase inhibitor.

### Example 23.

Effect of the compounds of present invention in Bcr-Abl mutated imatinib mesylate-resistant cell lines on cell cycle and apoptosis using Flow cytometry.

Cells for flow cytometry were seeded at a density of 10 X 10⁴ cells/mL and incubated with the test compounds/imatinib mesylate (5 µM) for 24 h at 37 °C in 5% CO₂ incubator. At the end of incubation, cells were harvested by centrifugation at 1000 rpm for 10 minutes, followed by 2 washes with phosphate buffered saline (PBS). Cell pellet from the last wash was gradually resuspended in 70 % ice-cold ethanol that facilitates the permeabilisation of stains. Cell suspension was stored for a minimum period of 4 h before staining with propidium iodide (PI). Fixed cells were stained with PI (80 µg/mL) in presence of RNase A (50 µg/mL), and read on BD FACS caliber for cell cycle analysis. The results of this study are presented in Figure 4.

**Conclusion:** Induction of apoptosis induced by the compounds of the present invention in imatinib mesylate-resistant cell lines is significant.

### Example 24

*In-vivo* efficacy testing of the compounds of the present invention in imatinib mesylate-resistant and imatinib mesylate-sensitive tumor models was studied by using cell lines such as Ba/F3 transfectants expressing full-length wild type Bcr-Abl (Ba/F3 Bcr-Abl/WT) or mutated Bcr-Abl (Ba/F3 Bcr-Abl/T315I).

### Objective

*In- vivo* efficacy testing of caffeic acid derivatives in imatinib mesylate resistant and imatinib mesylate sensitive tumor models.

### Cell Lines

Cell lines Ba/F3 transfectants expressing full-length wild type imatinib mesylate sensitive (Ba/F3 Bcr-Abl/WT) or mutated imatinib mesylate resistant (Ba/F3 Bcr-Abl/T315I) were used in this study. These recombinant cell lines were licensed from Dr. Brian Druker's laboratory, Howard Hughes Medical Institute, Oregon Health and Science University Cancer Institute, Portland, Oregon, USA. (Cancer Research, 2002, 62, 7149-7153).

### Compound storage

All the compounds including standard were stored at 4-8 °C in an amber colored bottle. The compounds in solutions were also maintained at 4-8 °C in a refrigerator. Sample for animal injection was made fresh everyday, residual volume were pooled and discarded as per standard operating procedure (SOP) for chemical disposals.

### Dose preparation

Required compound was weighed and admixed with 0.5 % (w/v) carboxymethylcellulose (CMC) and triturated with Tween-20 (*secundum artum*) with gradual addition of water to make up the final concentration.

### Efficacy study in SCID mice

A group of 110 Severely Combined Immune-Deficient (SCID strain-CBySmn.CB17-*Prkdc^{scid}*/J, *The Jackson Laboratory, Stock # 001803)* male mice, 6-9 weeks old, weighing ~20g, were used.

Ba/F3 Bcr-Abl/WT cells and Ba/F3 Bcr-Abl/T315I cells were grown in RPMI1640 medium containing 10% fetal calf serum in 5%CO₂ incubator at 37°C. Cells were pelleted by centrifugation at 1000-rpm for 10 minutes. Cells were resuspended in saline to get a count of 80-100 X 10⁶ cells per mL, 0.2 mL of this cell suspension was injected by subcutaneous (s.c.) route in SCID mice. Mice were observed alternate days for palpable tumor mass. Once the tumor size reached a size of 5-7 mm in diameter, animals were randomized into respective treatment groups. Dose of control or test compound was administered every day. Tumor size was recorded at 2-5 day intervals. Tumor weight (mg) was estimated according to the formula for a prolate ellipsoid: {Length (mm) x [width (mm) ²] x 0.5} assuming specific gravity to be one and π to be three. Tumor growth in compound treated animals is calculated as T/C (Treated/Control) x 100% and Growth inhibition Percent (GI%) was [100-T/C%]. Respective treatment groups are presented in Table 6.

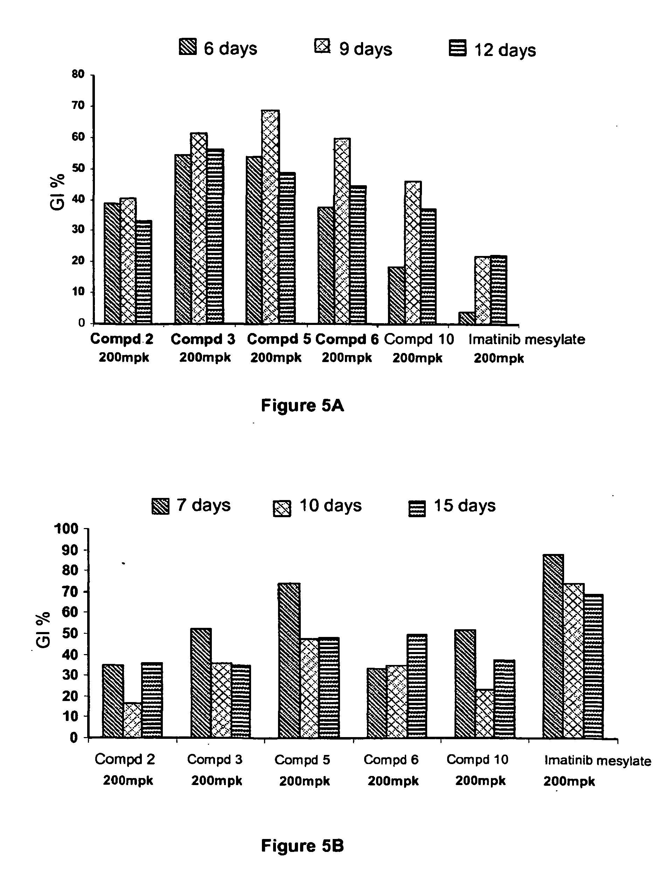

Results are graphically presented in Figure 5A and Figure 5B.

**Table 6: Treatment groups in the xenograft models (SET I and SET II) (SET I) Designation: Ba/F3 Bcr-Abl/T315I**

| **Groups** | **Sample** | **Dose** | **Route** | **Number of treatments** | **n=** |
|---|---|---|---|---|---|
| I | Control (untreated) | Vehicle | p.o. | q1d X 12 | 8 |
| II | Compound. 2 | 200 mg/kg | p.o. | q1d X 12 | 8 |
| III | Compound 3 | 200 mg/kg | p.o. | q1d X 12 | 8 |
| IV | Compound 5 | 200 mg/kg | p.o. | q1d X 12 | 8 |
| V | Compound 6 | 200 mg/kg | p.o. | q1d X 12 | 8 |
| VI | Compound 10 | 200 mg/kg | p.o. | q1d X 12 | 7 |
| VII | Imatinib Mesylate | 200 mg/kg | p.o. | q1d X 12 | 7 |

| | | | | | |
|---|---|---|---|---|---|
| p.o. = per oral; n = number of animals q1dx12 = single administration for 12 days | | | | | |

**(SET II) Designation: Ba/F3 Bcr-Abl/WT**

| **Groups** | **Sample** | **Dose** | **Route** | **Number of treatments** | **n=** |
|---|---|---|---|---|---|
| I | Control (untreated) | Vehicle | p.o. | q1d X 14 | 8 |
| II | Compound 2 | 200 mg/kg | p.o. | q1d X 14 | 8 |
| III | Compound 3 | 200 mg/kg | p.o. | q1d X 14 | 8 |
| IV | Compound. 5 | 200 mg/kg | p.o. | q1d X 14 | 8 |
| V | Compound. 6 | 200 mg/kg | p.o. | q1d X 14 | 8 |
| VI | Compound 10 | 200 mg/kg | p.o. | q1d X 14 | 8 |
| VII | Imatinib Mesylate | 200 mg/kg | p.o. | q1d X 14 | 7 |

| | | | | | |
|---|---|---|---|---|---|
| p.o. = per oral; n = number of animals q1dx14 = single administration for 14 days | | | | | |

Conclusion: The data presented in Figure 5A and Figure 5B demonstrates that the compounds of the present invention exhibited significantly greater *in-vivo* efficacy than imatinib mesylate in inhibiting the most predominant mutated form of Bcr-Abl i.e. Ba/F3 Bcr-Abl/T315I when tested at the same doses as that of wild type Bcr-Abl expressing xenograft i.e. Ba/F3 Bcr-Abl/WT.

It should be noted that, as used in this specification and the appended claims, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

All publications and patent applications in this specification are indicative of the level of ordinary skill in the art to which this invention pertains.

## Claims

1. A derivative of caffeic acid represented by the general formula (1) 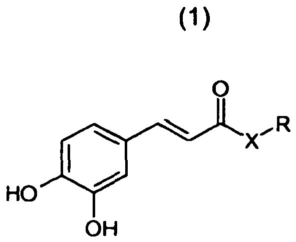 wherein
X is O, NH, or heterocyclyl;
R is a straight chain or branched alkyl which is optionally substituted, aryl which is optionally substituted, heterocyclyl which is optionally substituted, or absent;
in all its stereoisomeric and tautomeric forms, and mixtures thereof in all ratios or a pharmaceutically acceptable salt thereof; for use in the treatment of chronic myeloid leukemia (CML) that is resistant to treatment with Gleevec (Imatinib mesylate).

2. The derivative of caffeic acid for use according to claim 1 which is selected from:
3-(3,4-Dihydroxy-phenyl)-acrylic acid methyl ester;
3-(3,4-Dihydroxy-phenyl)-acrylic acid ethyl ester;
(*E*)-3-(3,4-dihydroxyphenyl)-acrylic acid 2-nitro-ethyl ester;
3-(3,4-Dihydroxy-phenyl)-acrylic acid *n*-propyl ester;
3-(3,4-Dihydroxy-phenyl)-acrylic acid *i*-propyl ester;
3-(3,4-Dihydroxy-phenyl)-acrylic acid butyl ester;
(*E*)-3-(3,4-Dihydroxy-phenyl)-1-piperidin-1-yl-propenone;
3-(3,4-Dihydroxy-phenyl)-1-(4-ethyl-piperazin-1-yl)-propenone;
1-(4-Benzyl-piperazin-1-yl)3-(3,4-dihydroxy-phenyl)-propenone;
(*E*)-3-{4-[3-(3,4-Dihydroxy-phenyl)-acryloyl]-piperazin-1-yl} propionitrile;
(*E*)-3-(3,4-Dihydroxyphenyl)-1-[4-(1-methyl-piperidin-4-yl) piperazin-1-yl]- propenone;
(*E*)-3-(3,4-Dihydroxyphenyl)-1-(4-phenylpiperazin-1-yl)-propenone;
(*E*)-1-(4-Acetyl-piperazin-1-yl)-3-(3,4-dihydroxy-phenyl)-propenone;
(*E*)-3-(3,4-Dihydroxy-phenyl)-1-(4-phenethyl-piperazin-1-yl)-propenone;
(*E*)-3-(3,4-Dihydroxy-phenyl)-1-morpholin-4-yl-propenone;
3-(3,4-Dihydroxy-phenyl)-N-(3-dimethylamino-propyl)-acrylamide;
(*E*)-3-(3,4-Dihydroxyphenyl)-N-isopropyl-acrylamide; or
1-(4-Benzyl-piperidin-1-yl)-3-(3,4-dihydroxy-phenyl)-propenone.

3. The derivative of caffeic acid for use according to claim 2 which is selected from:
3-(3,4-Dihydroxy-phenyl)-acrylic acid methyl ester;
3-(3,4-Dihydroxy-phenyl)-acrylic acid ethyl ester;
3-(3,4-Dihydroxy-phenyl)-acrylic acid *n*-propyl ester;
3-(3,4-Dihydroxy-phenyl)-acrylic acid *i*-propyl ester;
1-(4-Benzyl-piperazin-1-yl)-3-(3,4-dihydroxy-phenyl)-propenone;
(*E*)-3-(3,4-Dihydroxyphenyl)-N-isopropyl-acrylamide; or
1-(4-Benzyl-piperidin-1-yl)-3-(3,4-dihydroxy-phenyl)-propenone.

4. The derivative of caffeic acid for use according to claim 1, wherein the resistance to treatment with Gleevec (Imatinib mesylate) is caused by Bcr-Abl mutation.

5. The derivative of caffeic acid for use according to claim 1, wherein the resistance to treatment with Gleevec (Imatinib mesylate) arises due to a mutation found in a cell selected from K-562-R, 32Dcl Bcr-Abl T315l, Ba/F3 Bcr-Abl/T315I, Ba/F3 Bcr-Abl/E255K, Ba/F3 Bcr-Abl/H396P, Ba/F3 Bcr-Abl/M351T, Ba/F3 Bcr-Abl/F359V, Ba/F3 Bcr-Abl/E255V, Ba/F3 Bcr-Abl/F317L, Ba/F3 Bcr-Abl/H396R, Ba/F3 Bcr-Abl/M244V, Ba/F3 Bcr-Abl/Q252H, Ba/F3 Bcr-Abl/Y253F, or Ba/F3 Bcr-Abl/Y253H cell, line.

## Patentansprüche

1. Ein Derivat von Kaffeesäure, repräsentiert durch die allgemeine Formel (1) 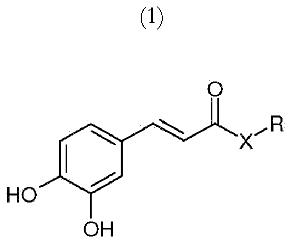 wobei
X O, NH oder Heterocyclyl ist;
R ein geradkettiges oder verzweigtes Alkyl, das optional substituiert ist, Aryl, das optional substituiert ist, Heterocyclyl, das optional substituiert ist, oder nicht vorhanden ist;
in allen stereoisomeren oder tautomeren Formen, und Mischungen davon in allen Verhältnissen oder pharmazeutisch annehmbare Salze davon;
zur Verwendung in der Behandlung von chronischer myeloider Leukämie (CML), die gegenüber Behandlung mit Gleevec (Imatinib-mesylat) resistent ist.

2. Das Derivat von Kaffeesäure zur Verwendung gemäß Anspruch 1, das ausgewählt ist aus:
3-(3,4-Dihydroxyphenyl)-acrylsäuremethylester;
3-(3,4-Dihydroxyphenyl)-acrylsäureethylester;
(*E*)-3-(3,4-Dihydroxyphenyl)-acrylsäure-2-nitroethylester;
3-(3,4-Dihydroxyphenyl)-acrylsäure-n-propylester;
3-(3,4-Dihydroxyphenyl)-acrylsäure-i-propylester;
3-(3,4-Dihydroxyphenyl)-acrylsäure-butylester;
(*E*)-3-(3,4-Dihydroxyphenyl)-1-piperidin-1-ylpropenon;
3-(3,4-Dihydroxyphenyl)-1-(4-ethylpiperazin-1-yl)-propenon;
1-(4-Benzylpiperazin-1-yl)-3-(3,4-dihydroxyphenyl)-propenon;
(*E*)-3-{4-3-(3,4-Dihydroxyphenyl)-acryloyl]-piperazin-1-yl} -propionitril;
(*E*)-3-(3,4-Dihydroxyphenyl)-1-[4-(1-methylpiperidin-4-yl)-piperazin-1-yl]-propenon;
(*E*)-3-(3,4-Dihydroxyphenyl)-1-(4-phenylpiperazin-1-yl)-propenon;
(*E*)-1-(4-Acetylpiperazin-1-yl)-3-(3,4-dihydroxyphenyl)-propenon;
(*E*)-3-(3,4-Dihydroxyphenyl)-1-(4-phenethylpiperazin-1-yl)-propenon;
(*E*)-3-(3,4-Dihydroxyphenyl)-1-morpholino-4-ylpropenon;
3-(3,4-Dihydroxyphenyl)-N-(3-dimethylaminopropyl)-acrylamid;
(*E*)-3-(3,4-Dihydroxyphenyl)-N-isopropylacrylamid; oder
1-(4-Benzylpiperidin-1-yl)-3-(3,4-dihydroxyphenyl)-propenon.

3. Das Derivat von Kaffeesäure zur Verwendung gemäß Anspruch 2, das ausgewählt ist aus:
3-(3,4-Dihydroxyphenyl)-acrylsäuremethylester;
3-(3,4-Dihydroxyphenyl)-acrylsäureethylester;
3-(3,4-Dihydroxyphenyl)-acrylsäure-n-propylester;
3-(3,4-Dihydroxyphenyl)-acrylsäure-i-propylester;
1-(4-Benzylpiperazin-1-yl)-3-(3,4-dihydroxyphenyl)-propenon;
(*E*)-3-(3,4-Dihydroxyphenyl)-N-isopropylacrylamid; oder
1-(4-Benzylpiperidin-1-yl)-3-(3,4-dihydroxyphenyl)-propenon.

4. Das Derivat von Kaffeesäure zur Verwendung gemäß Anspruch 1, wobei die Resistenz gegen Behandlung mit Gleevec (Imatinib-mesylat) durch Bcr-Abl-Mutation verursacht wird.

5. Das Derivat von Kaffeesäure zur Verwendung gemäß Anspruch 1, wobei die Resistenz gegen Behandlung mit Gleevec (Imatinib-mesylat) auf Grund einer Mutation, die in einer Zelle ausgewählt aus den Zellinien K-562-R, 32Dcl Bcr-Abl T315I, Ba/F3 Bcr-Abl/T315I, Ba/F3 Bcr-Abl/E255K, Ba/F3 Bcr-Abl/H396P, Ba/F3 Bcr-Abl/M351T, Ba/F3 Bcr-Abl/F359V, Ba/F3 Bcr-Abl/E255V, Ba/F3 Bcr-Abl/F317L, Ba/F3 Bcr-Abl/H396R, Ba/F3 Bcr-Abl/M244V, Ba/F3 Bcr-Abl/Q252H, Ba/F3 Bcr-Abl/Y253F oder Ba/F3 Bcr-Abl/Y253H gefunden wird, entsteht.

## Revendications

1. Dérivé d'acide caféique représenté par la formule générale (1) : 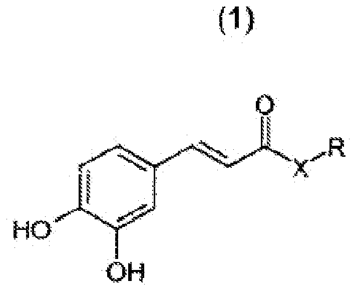 dans laquelle
X représente O, NH ou hétérocyclyle ;
R représente un alkyle à chaîne droite ou ramifié qui est facultativement substitué, aryle qui est facultativement substitué, hétérocyclyle qui est facultativement substitué, ou est absent ;
dans toutes ses formes stéréoisomères et tautomères, et leurs mélanges dans tous rapports ou un sel pharmaceutiquement acceptable de ce dérivé ; en vue d'une utilisation dans le traitement de la leucémie myéloïde chronique (CML) qui est résistante au traitement par le Gleevec (mésylate d'imatinib).

2. Dérivé d'acide caféique en vue d'une utilisation selon la revendication 1, qui est choisi parmi :
l'ester méthylique de l'acide 3-(3,4-dihydroxy-phényl)-acrylique ;
l'ester éthylique de l'acide 3-(3,4-dihydroxy-phényl)-acrylique ;
l'ester 2-nitro-éthylique de l'acide (*E*)-3-(3,4-dihydroxy-phényl)-acrylique ;
l'ester *n*-propylique de l'acide 3-(3,4-dihydroxy-phényl)-acrylique ;
l'ester *i*-propylique de l'acide 3-(3,4-dihydroxy-phényl)-acrylique ;
l'ester butylique de l'acide 3-(3,4-dihydroxy-phényl)-acrylique ;
la (*E*)-3-(3,4-dihydroxy-phényl)-1-pipéridin-1-yl-propénone ;
la 3-(3,4-dihydroxy-phényl)-1-(4-éthyl-pipérazin-1-yl)-propénone ;
la 1-(4-benzyl-pipérazin-1-yl)-3-(3,4-dihydroxy-phényl)-propénone ;
le (*E*)-3-{4-[3-(3,4-dihydroxy-phényl)-acryloyl)]-pipérazin-1-yl}propionitrile ;
la (*E*)-3-(3,4-dihydroxyphényl)-1-[4-(1-méthyl-pipéridin-4-yl)pipérazin-1-yl]-propénone ;
la (*E*)-3-(3,4-dihydroxyphényl)-1-(4-phénylpipérazin-1-yl)-propénone ;
la (*E*)-1-(4-acétyl-pipérazin-1-yl)-3-(3,4-dihydroxy-phényl)-propénone ;
la (*E*)-3-(3,4-dihydroxy-phényl)-1-(4-phénéthyl-pipérazin-1-yl)-propénone ;
la (*E*)-3-(3,4-dihydroxy-phényl)-1-morpholin-4-yl-propénone ;
le 3-(3,4-dihydroxy-phényl)-N-(3-diméthylamino-propyl)-acrylamide ;
le (*E*)-3-(3,4-dihydroxyphényl)-N-isopropyl-acrylamide ; ou
la 1-(4-benzyl-pipéridin-1-yl)-3-(3,4-dihydroxy-phényl)-propénone.

3. Dérivé d'acide caféique en vue d'une utilisation selon la revendication 2, qui est choisi parmi :
l'ester méthylique de l'acide 3-(3,4-dihydroxy-phényl)-acrylique ;
l'ester éthylique de l'acide 3-(3,4-dihydroxy-phényl)-acrylique ;
l'ester *n*-propylique de l'acide 3-(3,4-dihydroxy-phényl)-acrylique ;
l'ester i-propylique de l'acide 3-(3,4-dihydroxy-phényl)-acrylique ;
la 1-(4-benzyl-pipérazin-1-yl)-3-(3,4-dihydroxy-phényl)-propénone ;
le (*E*)-3-(3,4-dihydroxyphényl)-N-isopropyl-acrylamide ; ou
la 1-(4-benzyl-pipéridine-1-yl)-3-(3,4-dihydroxy-phényl)-propénone.

4. Dérivé d'acide caféique en vue d'une utilisation selon la revendication 1, dans lequel la résistance au traitement par le Gleevec (mésylate d'imatinib) est provoquée par une mutation Bcr-Abl.

5. Dérivé d'acide caféique en vue d'une utilisation selon la revendication 1, dans lequel la résistance au traitement par le Gleevec (mésylate d'imatinib) se produit en raison d'une mutation trouvée dans une cellule choisie parmi les lignées cellulaires K-562-R, 32Dcl Bcr-Abl T3151, Ba/F3 Bcr-Abl/T315I, Ba/F3 Bcr-Abl/E255K, Ba/F3 Bcr-Abl/H396P, Ba/F3 Bcr-Abl/M351T, Ba/F3 Bcr-Abl/F359V, Ba/F3 Bcr-Abl/E255V, Ba/F3 Bcr-Abl/F317L, Ba/F3 Bcr-Abl/H396R, Ba/F3 Bcr-Abl/M244V, Ba/F3 Bcr-Abl/Q252H, Ba/F3 Bcr-Abl/Y253F ou Ba/F3 Bcr-Abl/Y253H.
